# FASCICULE DE BREVET EUROPEEN

(11) **EP 2 440 511 B1**
(45) Date de publication et mention de la délivrance du brevet: **17.10.2018**
(21) Numéro de dépôt: 10737971.1
(22) Date de dépôt: 08.06.2010
(51) Int. Cl.: C07C 37/02, C07B 41/02, C07C 41/26, C07C 45/64, C07C 205/22, C07C 39/27, C07C 39/04, C07C 49/825, C07C 255/53, C07C 253/30, C07C 201/12

(54) **PROCÉDÉ D' HYDROXYLATION DE COMPOSÉS ARYLES HALOGÉNÉS**
VERFAHREN ZUR HYDROXYLIERUNG VON HALOGENIERTEN ARYLVERBINDUNGEN
METHOD FOR THE HYDROXYLATION OF HALOGENATED ARYL COMPOUNDS

(30) Priorité: 08.06.2009 FR 0902767
(43) Date de publication de la demande: 18.04.2012
(73) Titulaire: Centre National de la Recherche Scientifique (C.N.R.S.), 75016 Paris (FR)
(72) Inventeur: TAILLEFER, Marc, F-34570 Vailhauques (FR); TLILI, Anis, F-34000 Montpellier (FR); MONNIER, Florian, F-34070 Montpellier (FR); XIA, Ning, F-67400 Illkirch-Grafferstaden (FR)
(74) Mandataire: Lavoix
(86) Numéro de dépôt international: PCT/FR2010/051140
(87) Numéro de publication internationale: WO 2010/142913

(56) Documents cités:
- JP-B2- 3 278 945
- US-A- 2 041 592
- KORMOS C M ET AL: "Direct conversion of aryl halides to phenols using high-temperature or near-critical water and microwave heating" TETRAHEDRON, ELSEVIER SCIENCE PUBLISHERS, AMSTERDAM, NL, vol. 62, no. 19, 8 mai 2006 (2006-05-08), pages 4728-4732, XP025001922 ISSN: 0040-4020 [extrait le 2006-05-08] cité dans la demande
- ZHAO D. ET AL.: "Synthesis of phenol, aromatic ether, and benzofuran derivatives by copper-catalysed hydroxylation of aryl halides" ANGEWANDTE CHEMIE INTERNATIONAL EDITION ENGLISH, vol. 48, 2 novembre 2010 (2010-11-02), pages 8729-8732, XP002569082
- COMDOM ROLANDO F PELLON ET AL: "Synthesis of salicylic acid derivatives from the corresponding 2- chlorobenzoic acid using water as solvent", SYNTHETIC COMMUNICATIONS, TAYLOR & FRANCIS INC, PHILADELPHIA, PA; US, vol. 32, no. 13, 1 January 2002 (2002-01-01), pages 2055-2059, XP002472756, ISSN: 0039-7911, DOI: 10.1081/SCC-120004857

## Description

La présente invention est définie dans les revendications et concerne un procédé d'hydroxylation de composés aryles halogénés en présence d'un ligand. Il s'agit en particulier d'un procédé d'hydroxylation de composés aryles iodés ou bromés en présence d'un ligand pour obtenir des phénols ou des éthers diaryles.

Les phénols sont utilisés dans de nombreux domaines d'application, comme la santé humaine, animale ou végétale, les matériaux ou encore l'optique non linéaire.

Les réactions d'hydroxylation de composés aryles halogénés sont connues comme une des voies de synthèse des phénols.

Le document « Practical Imidazole-Based Phosphine Ligands for Selective Palladium-Catalyzed Hydroxylation of Aryl Halides », Beller et Al, Angewandte Chemie International Edition, 2009, 48, 918-921, divulgue des réactions d'hydroxylation de composés aryles halogénés en présence d'un catalyseur au Palladium et de ligands phosphine complexes à base phosphine d'imidazole.

Le document « the Selective Reaction of Aryl Halides with KOH : Synthesis of Phenols, Aromatic Ethers and Benzofurans», Buchwald et Al, Journal of The American Chemical Society, 2006, 128, 10694-10695, divulgue également une réaction d'hydroxylation de composés aryles halogénés en présence d'un catalyseur au Palladium et d'un ligand complexe à base diaryle.

Ces systèmes catalytiques sont toxiques en raison de la présence du palladium et coûteux à mettre en oeuvre. Le document « Direct conversion of aryl halides to phénols using high-temperature or near-critical water and microwave heating », C.M Kormos et N.E Leadbeater, Tetrahedron, 2006, 62, 4728-4732 divulgue l'utilisation d'un système catalytique associant le Iodure de cuivre et la L-Proline dans une réaction d'hydroxylation de composés aryles iodés ou bromés en milieu aqueux.

Cependant, la réaction décrite dans ce document nécessite des températures élevées, supérieures à 200°C, ces températures étant atteintes par un système de chauffage aux micro-ondes ; de plus les rendements obtenus pour la synthèse des phénols restent relativement faibles. Le document JP 3 278945 décrit aussi un procédé d'hydroxylation de composés aryles halogénés en présence d'un catalyseur à base de cuivre et de 8-hydroxyquinoléine.

Ainsi, un premier objectif de la présente invention consiste à proposer un procédé d'hydroxylation de composés aryles halogénés, qui s'affranchisse des inconvénients connus de l'état de la technique mentionnés ci-dessus.

Un autre objectif de la présente invention est de proposer un procédé économique, facilement industrialisable et peu toxique permettant l'hydroxylation de composés aryles halogénés, dans des conditions douces,

Un autre objectif encore consiste à proposer un procédé d'hydroxylation de composés aryles halogénés avec des rendements élevés.

Comme autre objectif, la présente invention vise à proposer un procédé d'hydroxylation de composés aryles halogénés de mise en oeuvre aisée, facilement industrialisable, et adaptable à une grande variété de substrats, sans modification importante des conditions opératoires.

La présente invention a pour objet un procédé d'hydroxylation de composés aryles halogénés, mis en oeuvre à une température inférieure à 150° C, en présence d'un système catalytique consistant en un catalyseur à base de cuivre et un ligand L selon le schéma réactionnel suivant : 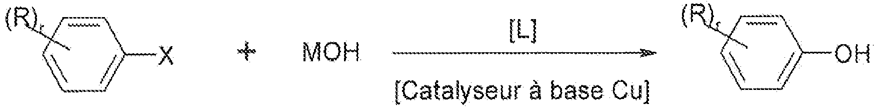
◆ R étant choisi parmi les groupes à effet inductif accepteur et les groupes à effet mésomère donneur définis dans les revendications;
◆ M étant choisi parmi les cations alcalins ou alcalinoterreux ;
◆ X étant un atome d'halogène ;
◆ r étant compris entre 0 et 5 ;
◆ L représente un composé de formule I 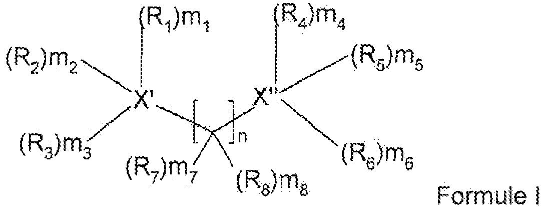 dans laquelle
◆ X', X", identiques ou différents sont choisis parmi l'atome d'azote, le groupe C=O, le groupe C=S et le groupe C-OH ;
◆ m₁ à m₈ identiques ou différents représentent 0 ou 1 ;
◆ n représente 1 ou 2 ;
◆ R₁, R₂, R₃, R₄, R₅, R₆, R₇ et R₈ identiques ou différents sont choisis parmi :
   - un atome d'hydrogène ;
   - un groupe hydrocarboné ramifié, linéaire, monocyclique ou polycyclique, comportant de 1 à 20 atomes de carbone, et pouvant comporter une ou plusieurs insaturations sous forme de double(s) et/ou triple(s) liaison(s), de préférence méthyle, isobutyle, phényle ;
   - une amine primaire, secondaire ou tertiaire -NR'R", avec R' et R" identiques ou différents représentant un groupe alkyle en C₁ à C₁₀, de préférence en C₁ à C₆, linéaire ou ramifié, de préférence méthyle ;
   - un groupe hydroxyle ;
◆ ou R₄, R₅, X" et R₇ forment un groupe hétérocyclique, de préférence pyrrolidine ;
◆ ou R₁, X' et R₇ forment un groupe phénol ;
◆ ou R₁, X', X", R₇ et R₄ forment un groupe polycyclique constitué par au moins 3 cycles saturés et/ou insaturés ou par au moins 3 cycles dont l'un seul d'entre eux ou deux d'entre eux est (sont) aromatique(s) et formant entre eux des systèmes ortho- ou ortho- et peri-condensés, de préférence le groupe phénanthroline.
Dans un mode de réalisation particulier, le ligand L est choisi parmi les composés de formule I
dans laquelle
◆ X', X", identiques ou différents sont choisis parmi l'atome d'azote, le groupe C=O, le groupe C=S et le groupe C-OH ;
◆ m₁ à m₈ identiques ou différents représentent 0 ou 1 ;
◆ n représente 1 ou 2 ;
◆ R₁, R₂, R₃, R₄, R₅, R₆, R₇ et R₈ identiques ou différents sont choisis parmi :
   - un atome d'hydrogène ;
   - un groupe hydrocarboné ramifié, linéaire, monocyclique ou polycyclique, comportant de 1 à 20 atomes de carbone, et pouvant comporter une ou plusieurs insaturations sous forme de double(s) et/ou triple(s) liaison(s), de préférence méthyle, isobutyle, phényle ;
   - une amine primaire, secondaire ou tertiaire -NR'R", avec R' et R" identiques ou différents représentant un groupe alkyle en C₁ à C₁₀, de préférence en C₁ à C₆, linéaire ou ramifié, de préférence méthyle ;
   - un groupe hydroxyle ;
◆ ou R₄, X" et R₇ forment un groupe hétérocyclique, de préférence pyrrolidine ;
◆ ou R₁, X' et R₇ forment un groupe phénol ;
◆ ou R₁, X', X", R₇ et R₄ forment un groupe polycyclique constitué par au moins 3 cycles saturés et/ou insaturés ou par au moins 3 cycles dont l'un seul d'entre eux ou deux d'entre eux est (sont) aromatique(s) et formant entre eux des systèmes ortho- ou ortho- et peri-condensés, de préférence le groupe phénanthroline.

Dans un mode de réalisation particulier le ligand L est choisi parmi les ligands de formule générale I dans laquelle :
◆ X', X", identiques ou différents sont choisis dans le groupe constitué par l'atome d'azote, le groupe C=O, le groupe C=S et le groupe C-OH ;
◆ m₁ à m₈ identiques ou différents représentent 0 ou 1 ;
◆ n représente 1 ou 2 ;
◆ R₁, R₂, R₃, R₄, R₅, R₆, R₇ et R₈ identiques ou différents sont choisis parmi :
   - un atome d'hydrogène ;
   - un groupe alkyle, en C1 à C10, de préférence en C1 à C6, linéaire ou ramifié, de préférence méthyle, terbutyle ;
   - un groupe aryle, de préférence phényle ;
   - une amine primaire, secondaire ou tertiaire -NR'R", avec R' et R" identiques ou différents représente un atome d'hydrogène, un groupe alkyle en C1 à C10, de préférence en C1 à C6, linéaire ou ramifié, de préférence méthyle ;
   - un groupe hydroxyle ;
◆ R₇ et R₈ identiques ou différents représentent un atome d'hydrogène ;
◆ ou R₄, R₅, X" et R₇ forment un groupe hétérocyclique, de préférence pyrrolidine ;
◆ ou R₁, X' et R₇ forment un groupe phénol ;
◆ ou R₁, X', X", R₇ et R₄ forment un groupe polycyclique constitué par au moins 3 cycles saturés et/ou insaturés ou par au moins 3 cycles dont l'un seul d'entre eux ou deux d'entre eux est (sont) aromatique(s) et formant entre eux des systèmes ortho- ou ortho- et peri-condensés, de préférence le groupe phénanthroline.

Dans un mode de réalisation particulier, le ligand L est choisi parmi les ligands de formule générale I dans laquelle :
◆ X', X", identiques ou différents sont choisis parmi l'atome d'azote, le groupe C=O, le groupe C=S et le groupe C-OH ;
◆ m₁ à m₈ identiques ou différents représentent 0 ou 1 ;
◆ n représente 1 ou 2 ;
◆ R₁, R₂, R₃, R₄, R₅, R₆, R₇ et R₈ identiques ou différents sont choisis parmi :
   - un atome d'hydrogène ;
   - un groupe alkyle, en C₁ à C₁₀, de préférence en C₁ à C₆, linéaire ou ramifié, de préférence méthyle, terbutyle ;
   - un groupe aryle, de préférence phényle ;
   - une amine primaire, secondaire ou tertiaire -NR'R", avec R' et R" identiques ou différents représentant un atome d'hydrogène, un groupe alkyle en C1 à C10, de préférence en C1 à C6, linéaire ou ramifié, de préférence méthyle ;
   - un groupe hydroxyle ;
◆ R₇ et R₈ identiques ou différents représentent un atome d'hydrogène ; ou
◆ R₄, X" et R₇ forment un groupe hétérocyclique, de préférence pyrrolidine ; ou
◆ R₁, X' et R₇ forment un groupe phénol ; ou
◆ R₁, X', X", R₇ et R₄ forment un groupe polycyclique constitué par au moins 3 cycles saturés et/ou insaturés ou par au moins 3 cycles dont l'un seul d'entre eux ou deux d'entre eux est (sont) aromatique(s) et formant entre eux des systèmes ortho- ou ortho- et peri-condensés, de préférence le groupe phénanthroline.
Dans un mode de réalisation particulier, le ligand L est choisi parmi les composés de formule I 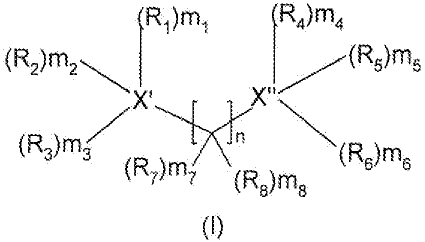 dans laquelle
◆ X', X", identiques ou différents sont choisis parmi l'atome d'azote, le groupe C=O, le groupe C=S et le groupe C-OH ;
◆ n représente 1 ;
◆ m₄, m₇ et m₈ représentent 1 ; m₁, m₂, m₃, m₅ et m₆ représentent 0 ou 1 ;
◆ R₁, R₂, R₃, R₅, R₆ et identiques ou différents sont choisis parmi :
   - un atome d'hydrogène ;
   - un groupe hydrocarboné ramifié, linéaire ou cyclique (mono- ou polycyclique), comportant de 1 à 20 atomes de carbone, et pouvant comporter une ou plusieurs insaturations sous forme de double(s) et/ou triple(s) liaison(s), de préférence méthyle, isobutyle, phényle ;
   - une amine primaire, secondaire ou tertiaire -NR'R", avec R' et R" identiques ou différents représentant un groupe alkyle en C₁ à C₁₀, de préférence en C₁ à C₆, linéaire ou ramifié, de préférence méthyle ;
   - un groupe hydroxyle ;
◆ R₈ représente H et R₄, X" et R₇ forment un groupe hétérocyclique, de préférence pyrrolidine ; ou
◆ X', X", identiques ou différents sont choisis parmi l'atome d'azote, le groupe C=O, le groupe C=S et le groupe C-OH ;
◆ n représente 1 ;
◆ m₂ à m₆ représentent 0 ou 1 ; m₁, m₇ et m₃ représentent 1 ;
◆ R₁ à R₆, identiques ou différents, sont choisis parmi :
   - un atome d'hydrogène ;
   - un groupe hydrocarboné ramifié, linéaire ou cyclique (mono- ou polycyclique), comportant de 1 à 20 atomes de carbone, et pouvant comporter une ou plusieurs insaturations sous forme de double(s) et/ou triple(s) liaison(s), de préférence méthyle, isobutyle, phényle ;
   - une amine primaire, secondaire ou tertiaire -NR'R", avec R' et R" identiques ou différents représentant un groupe alkyle en C₁ à C₁₀, de préférence en C₁ à C₆, linéaire ou ramifié, de préférence méthyle ;
   - un groupe hydroxyle ;
◆ R₁, X' et R₇ forment un groupe phénol et R₈ représente H; ou
◆ X', X", identiques ou différents sont choisis parmi l'atome d'azote, le groupe C=O, le groupe C=S et le groupe C-OH ;
◆ n représente 2 ;
◆ m₂, m₃ m₅, m₆ et m₈ représentent 0 ou 1 ; m₁, m₄ et m₇ représentent 1 ;
◆ R₂, R₃, R₅, R₆ et R₈, identiques ou différents, sont choisis parmi :
   - un atome d'hydrogène ;
   - un groupe hydrocarboné ramifié, linéaire ou cyclique (mono- ou polycyclique), comportant de 1 à 20 atomes de carbone, et pouvant comporter une ou plusieurs insaturations sous forme de double(s) et/ou triple(s) liaison(s), de préférence méthyle, isobutyle, phényle ;
   - une amine primaire, secondaire ou tertiaire -NR'R", avec R' et R" identiques ou différents représentant un groupe alkyle en C₁ à C₁₀, de préférence en C₁ à C₆, linéaire ou ramifié, de préférence méthyle ;
   - un groupe hydroxyle ;
◆ R₁, X', X", R₇ et R₄ forment un groupe polycyclique comprenant au moins 3 cycles saturés et/ou insaturés ou au moins 3 cycles dont l'un ou deux d'entre eux sont aromatiques et forment entre eux des systèmes ortho- ou ortho- et peri-condensés, de préférence le groupe phénanthroline.

Dans un mode de réalisation particulier le ligand L est choisi parmi les ligands de formule générale I dans laquelle X' et X" tous deux identiques représentent le groupe C=O, et
◆ m₂, m₃, m₅ et m₆ sont nuls
◆ m₁ et m₄ sont égaux à 1,
les m₁, m₂ et m₃ ainsi que R₁, R₂, R₃ ainsi que m₄, m₅, m₆ et R₄, R₅, R₆ étant tous interchangeables.
◆ m₇ et m₈ représentent 0 ou 1
◆ R₁ et R₄ identique ou différents sont choisis parmi ;
   - un groupe alkyle en C₁ à C₁₀, de préférence en C₁ à C₆, linéaire ou ramifié, de préférence méthyle, terbutyle ;
   - un groupe aryle, de préférence phényle ;
   - une amine primaire, secondaire ou tertiaire -NR'R", avec R' et R" identiques ou différents représentant un atome d'hydrogène, un groupe alkyle en C₁ à C₁₀, de préférence en C₁ à C₆, linéaire ou ramifié, de préférence méthyle ;
◆ R₇ et R₈ représentent un atome d'hydrogène.

Dans un mode de réalisation particulier le ligand L est choisi parmi les ligands de formule générale I dans laquelle X' représente un groupe C=O et X" représente l'atome d'azote, ou X' représente l'atome d'azote et X" représente un groupe C=O, et
◆ dans le cas où X' = C=O : m₂, m₃, m₆, m₈ sont nuls, m₁, m₄, m₅ et m₇ sont égaux à 1 et n est égal à 1; R₁ représente un groupe hydroxyle ;
   R₇ représente un atome d'hydrogène ;
   R₄, R₅, R₇ et X" forment un groupe pyrrolidine ;
◆ dans le cas où X" = C=O : m₅, m₆, m₃ et m₈ sont nuls, m₁, m₂, m₃, m₄ et m₇ sont égaux à 1 et n est égal à 1; R₄ représente un groupe hydroxyle
   R₇ représente un atome d'hydrogène ;
   R₁, R₂, R₇ et X' forment un groupe pyrrolidine ;

Dans un mode de réalisation particulier le ligand L est choisi parmi les ligands de formule générale I dans laquelle X' et X" représentent un atome d'azote, et
◆ n égal à 2;
◆ m₃ et m₆ sont nuls, m₁, m₂, m₄, m₅, m₇ et m₈ sont égaux à 0 ou 1 ;
◆ R₁, R₂, R₄ et R₅ identique ou différents sont choisis parmi :
   - un atome d'hydrogène ;
   - un groupe alkyle en C₁ à C₁₀, de préférence C₁ à C₆, linéaire ou ramifié, de préférence méthyle;
◆ ou R₁, X', X", R₇ et R₄ forment un groupe polycyclique constitué par au moins 3 cycles saturés et/ou insaturés ou par au moins 3 cycles dont l'un seul d'entre eux ou deux d'entre eux est (sont) aromatique(s) et formant entre eux des systèmes ortho- ou ortho- et peri-condensés, de préférence le groupe phénanthroline.

Dans un mode de réalisation particulier le ligand L est choisi parmi les ligands de formule générale I dans laquelle X' représente le groupe C-OH et X" représente le groupe C=O, ou X' représente le groupe C=O et X" représente le groupe C-OH, et
◆ dans le cas où X' = C=O : m₂, m₃, m₅, m₆, m₈ sont nuls, m₁, m₄, et m₇ sont égaux à 1 et n est égal à 1; R₁ représente un groupe alkyle en C₁ à C₁₀, de préférence C₁ à C₆, linéaire ou ramifié, de préférence méthyle;
   R₄, R₇ et X" forment un groupe phénol ;
◆ dans le cas où X" = C=O : m₅, m₆, m₈, m₂, m₃, sont nuls, m₁, m₄ et m₇ sont égaux à 1 et n est égal à 1; R₄ représente un groupe alkyle en C₁ à C₁₀, de préférence C₁ à C₆, linéaire ou ramifié, de préférence méthyle;
   R₁, R₇ et X' forment un groupe phénol.

Dans un mode de réalisation le ligand L est représenté par la formule Il 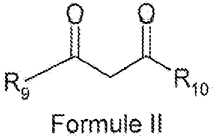 Où :
R₉, R₁₀ identiques ou différents représentent un groupe hydrocarboné ramifié, linéaire ou cyclique (mono- ou polycyclique), comportant de 1 à 20 atomes de carbones et pouvant comporter une ou plusieurs insaturations sous forme de double(s) et/ou triple(s) liaison(s), de préférence méthyle, isobutyle, phényle ; ou une amine primaire, secondaire ou tertiaire NR'R", avec R'R" identiques ou différents représentant un groupe alkyle en C₁ à C₁₀, de préférence en C₁ à C₆, linéaire ou ramifié, de préférence méthyle.

Dans un mode de réalisation le ligand L est représenté par la formule III 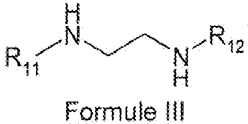 dans laquelle
R₁₁ et R₁₂, identiques ou différents représentent un atome d'hydrogène ou un groupe hydrocarboné ramifié, linéaire ou cyclique (mono- ou polycyclique), comportant de 1 à 20 atomes de carbones et pouvant comporter une ou plusieurs insaturations sous forme de double(s) et/ou triple(s) liaison(s), de préférence méthyle, isobutyle, phényle.

Dans un mode de réalisation, le ligand L est représenté par la formule IV 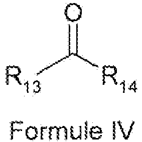 Où :
R₁₃ représente un groupe hydrocarboné ramifié, linéaire ou cyclique (mono- ou polycyclique), comportant de 1 à 20 atomes de carbones et pouvant comporter une ou plusieurs insaturations sous forme de double(s) et/ou triple(s) liaison(s), de préférence méthyle, isobutyle, phényle ; ou un groupe hydroxyle, et
R₁₄ représente un groupe hétérocyclique de préférence pyrrolidine ou un groupe phénol.

Dans un mode de réalisation préféré le ligand L est choisi parmi les composés suivants :

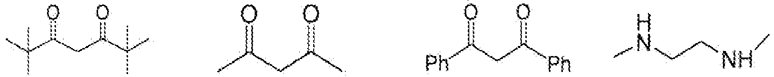

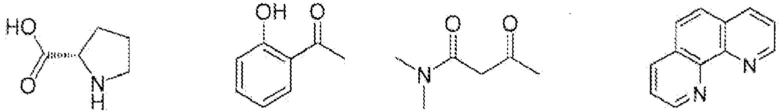

Dans un mode de réalisation le ligand L est :

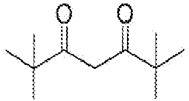

Dans un mode de réalisation le ligand L est :

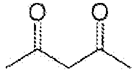

Dans un mode de réalisation le ligand L est :

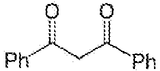

Dans un mode de réalisation le ligand L est :

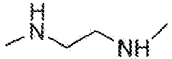

Dans un mode de réalisation le ligand L est :

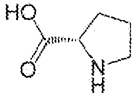

Dans un mode de réalisation le ligand L est :

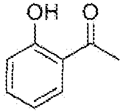

Dans un mode de réalisation le ligand L est :

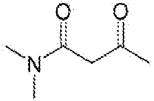

Dans un mode de réalisation le ligand L est :

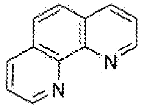

Les définitions suivantes sont valables pour l'ensemble de la description, sauf mention contraire :
« alkyle » ou « alkyl- » représente un radical hydrocarboné saturé, linéaire ou ramifié comportant de 1 à 10 atomes de carbone, de préférence de 1 à 6 atomes de carbone, et en particulier le radical méthyle, éthyle, les radicaux propyles, butyles, pentyles, hexyles, heptyles, octyles, nonyles et décyles ;
« aryle » ou « aryl- » représente un radical hydrocarboné aromatique mono ou polycyclique, et par exemple le radical phényle ou le radical naphtyle ;
« radical hydrocarboné » représente un radical hydrocarboné ramifié, linéaire ou cyclique (mono- ou polycyclique), comportant de 1 à 20 atomes de carbone, et pouvant comporter une ou plusieurs insaturations sous forme de double(s) et/ou triple(s) liaison(s), par exemple et de manière non limitative méthyle, éthyle, propyle, isopropyle, butyle, isobutyle, sec-butyle, tert-butyle, cyclohexyle, benzyle, phényle, vinyle, allyle, et autres ;
« hydroxyle» ou « hydroxy-» représente le groupe OH ;
« halogène » désigne fluor, chlore, brome et iode ;
« alkoxy » représente un groupe alkyle lié à un oxygène, alkyle ayant la définition précitée. A titre d'exemple on peut citer méthoxy, éthoxy etc.
« esters » représente un groupe -COOR, R étant choisi parmi les alkyles, alkyles ayant la définition précitée.
L'ensemble des radicaux dont les définitions figurent ci-dessus peuvent éventuellement être substitués par un ou plusieurs atomes d'halogènes, ou halogène a la définition précitée, par un ou plusieurs radicaux alkyle, par un ou plusieurs radicaux hydroxy, aryle, amino, les substituants pouvant être identiques ou différents.

Les catalyseurs à base de cuivre utilisés dans l'invention sont choisis parmi le cuivre métallique les oxydes de cuivre (I) ou cuivre (II), les hydroxydes de cuivre (I) ou cuivre (II), les sels inorganiques ou organiques de cuivre (I) ou cuivre (II) et les complexes de cuivre (I) ou cuivre (II) avec des ligands usuels.

Des exemples préférés de catalyseur à base de cuivre incluent, sans en être limité, le cuivre (0), les halogénures de cuivre (exemple : iodure de cuivre (I), bromure de cuivre (I), bromure de cuivre (II), chlorure de cuivre (I), chlorure de cuivre (II)), les oxydes ou hydroxydes de cuivre (exemple : oxyde de cuivre (I), oxyde de cuivre(II), hydroxyde de cuivre (II)), les nitrates de cuivre (exemple : nitrate de cuivre (I), nitrate de cuivre (II)), les sulfates ou sulfites de cuivre (exemple : sulfate de cuivre (I), sulfate de cuivre (II) sulfite de cuivre (I)) les sels organiques de cuivre dans lesquelles le contre ion présente au moins un atome de carbone (exemple : carbonate de cuivre (II) acétate de cuivre (I) acétate de cuivre (II), trifluorométhylsulfonate de cuivre (II), méthylate de cuivre (I), méthylate de cuivre (II), acétylacétonate de cuivre(II)).

Des catalyseurs préférés à base de cuivre sont le cuivre (0), l'iodure de cuivre (I) (CuI), l'oxyde de cuivre (II) (CuO), l'acétylacétonate de cuivre (II) [Cu(acac)₂], CuI + Cu(acac)₂.

Dans un mode de réalisation préféré, le catalyseur à base de cuivre est l'iodure de cuivre CuI.

Le procédé est mis en oeuvre à une température inférieure à 150°C.

Dans un mode de réalisation de l'invention, X est choisi entre brome et iode.

Dans un mode de réalisation particulier de l'invention X représente l'iode.

Dans ce mode de réalisation particulier, R est choisi parmi les groupes à effet inductif accepteur et les groupes à effet mésomère donneur.

Dans un mode de réalisation particulier de l'invention X représente le brome.

Dans ce mode de réalisation particulier, R est un groupe à effet inductif accepteur.

Dans un mode de réalisation particulier de l'invention le procédé est mis en oeuvre en deux étapes selon le schéma réactionnel suivant : 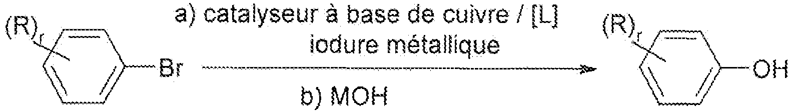 La première étape (a) correspond à une substitution nucléophile du bromobenzène par un iodure métallique en présence d'un catalyseur à base de cuivre et d'un ligand.
La deuxième étape correspond à la réaction d'hydroxylation de l'invention en présence d'un catalyseur à base de cuivre et d'un ligand.
Dans un mode de réalisation, le procédé en 2 étapes est mis en oeuvre pour R représentant un groupe à effet mésomère donneur.

Dans ce mode de réalisation le ratio molaire entre le nombre de moles d'iodure métallique et le nombre de moles de composé bromé varie entre 0,1 et 4, de préférence entre 1 et 3.

Dans un mode de réalisation de l'invention l'iodure métallique est choisi parmi Nal, KI ou CsI, de préférence l'iodure métallique est Nal

Le groupe à effet inductif accepteur est un groupe choisi dans le groupe constitué par le groupe NO₂, les esters, le groupe CN, un atome d'halogène, un groupe alkoxy.

Dans un mode de réalisation préféré, le groupe à effet inductif accepteur est choisi dans le groupe constitué par le groupe NO₂, le groupe CO₂Me, le groupe CN, l'atome de fluor, l'atome de brome, l'atome d'iode, l'atome de chlore, le groupe méthoxy.

Le groupe à effet mésomère donneur est un groupe choisi dans le groupe constitué par le groupe phényle, le groupe hydroxy (OH), un alkyle en C1 à C10, de préférence en C1 à C6, linéaire ou ramifié, un atome d'halogène, un atome d'hydrogène, un groupe alkoxy, ou R forme avec le phényle un dérivé naphtyl.

Dans un mode de réalisation préféré, le groupe à effet mésomère donneur est choisi dans le groupe constitué par le groupe phényle, le groupe hydroxy, le groupe méthyle, l'atome de fluor, l'atome d'hydrogène, le groupe méthoxy, ou R forme avec le phényle un dérivé naphtyl.

Dans un mode de réalisation préféré du procédé de l'invention, le cation M est choisi parmi potassium et césium. Le ratio molaire entre le nombre de moles de catalyseur à base de cuivre et le nombre de moles de composé arylé halogéné est préférentiellement compris entre 0,001 et 0,5, de préférence entre 0.05 et 0.2.
Le ratio molaire entre le nombre de moles de ligand L et le nombre de moles de composé arylé halogéné varie entre 0,001 et 0,9 ; de préférence entre 0,1 et 0,7 Dans un mode de réalisation le ratio molaire entre le nombre de moles de MOH et le nombre de moles de composé arylé halogéné varie entre 0,1 et 5, de préférence entre 2 et 4.

Dans un mode de réalisation le procédé de l'invention est mis en oeuvre en présence d'un solvant.

Le solvant est choisi dans le groupe constitué par l'eau, les solvants organiques et leurs mélanges.

Dans un mode de réalisation le solvant organique est choisi parmi :
- les carboxamides linéaires ou cycliques, de préférence, N-diméthylacetamide (DMAC), N,N-diéthylacetamide, diméthylformamide (DMF), diéthylformamide ou le 1-méthyl-2-pyrrolidinone (NMP) ;
- le diméthylsulfoxide (DMSO) ;
- l'hexaméthylphosphotriamide (HMPT) ;
- la tétraméthylurée;
- le benzène ;
- les composés à base nitro de préférence nitrométhane, nitroéthane, 1-nitropropane, 2-nitropropane ou nitrobenzene;
- les nitriles aliphatiques ou aromatiques de préférence acétonitrile, propionitrile, butanenitrile, isobutanenitrile, pentanenitrile, 2-methylglutaronitrile ou adiponitrile ;
- le tétraméthylène sulfone ;
- les carbonates organiques de préférence diméthylcarbonate, diisopropylcarbonate ou di-n-butylcarbonate ;
- les esters alkylés de préférence acétate d'éthyle ou d'isopropyl;
- les éthers aliphatiques ou aromatiques de préférence 1,4-dioxane ;
- les composés hydrocarbonés halogénés ou non halogénés de préférence toluène ou chlorobenzène ;
- les cétones de preference acétone, methyléthylcétone, methylisobutylcétone (MIBK), cyclopentanone, cyclohexanone;
- les hétérocycles comprenant un groupement azoté de préférence pyridine, picoline ou quinolines ;

### Seuls ou en mélange.

Dans un mode de réalisation préférée le solvant est un mélange eau et DMSO. Dans ce mode de réalisation le ratio eau :DMSO est compris entre 7 :1 et 1 :7, de préférence entre 1 :1 et 1 :3.

Dans un mode de réalisation préféré du procédé mis en oeuvre en 2 étapes, le solvant de l'étape a) est de préférence le 1,4-dioxane et le solvant de l'étape b) est l'eau.

Dans un mode de réalisation particulier, le temps de réaction est inférieur à 40 heures, de préférence compris entre 12 et 36 heures, de préférence entre 24 et 36 heures.

Dans un mode de réalisation particulier le temps de réaction de l'étape a), du procédé mis en oeuvre en 2 étapes, est inférieur à 10 heures.

Dans un mode de réalisation particulier le procédé mis en oeuvre par l'invention produit en outre un biaryléther (2), de préférence biphényléther. Ce mode de réalisation particulier correspond au schéma suivant : 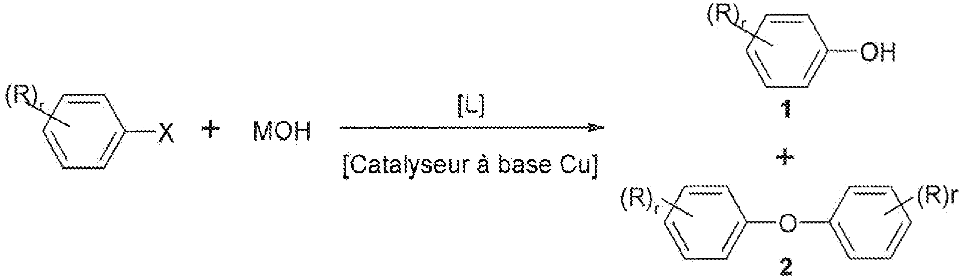 M, R, r et L ayant les définitions précitées.

Dans ce mode de réalisation, le catalyseur à base de cuivre est de préférence Cul, X représente de préférence l'atome d'iode et L est de préférence la ditertiobutylcétone, M, R et r ayant les définitions précitées.

La présente invention et ses différents modes de réalisation seront mieux compris à la lecture des exemples qui suivent. Ces exemples sont donnés à titre indicatif, sans caractère limitatif.

### Protocole opératoire général

Toutes les réactions sont mises en oeuvre dans des tubes de Schlenk de 35 ml ou dans un carrousel « Station de réaction RR98030 de tubes de Radley, sous une atmosphère d'azote pur et sec.

Le DMSO (diméthyle sulfoxide) est distillé et est stocké sur tamis moléculaire activé de 4Å sous atmosphère d'azote. Les autres solvants sont distillés et stockés sous atmosphère d'azote.

Le carbonate de césium (Alfa Aesar), l'hydroxyde de césium monohydraté (Alfa Aesar) et l'iodure de cuivre, CuI (Aldrich) et tous les autres matériaux solides sont stockés en présence de P4O10 dans un dessicateur sous vide à température ambiante. La 2,2, 6,6 - tetraméthyl -3,5 heptanedione à 98%+ (Alfa Aesar) et les autres ligands sont approvisionnés à partir des sources commerciales (Aldrich, Acros, Alfa Aesar, Fluka, Lancaster) et utilisés sans purification complémentaire.

Les Iodophényles et les Bromophényles sont approvisionnés à partir de sources commerciales. Si ce sont des solides ils sont recristallisés dans un solvant adapté. (Référence DD Perrin, W.L.F.Amarego, D.R. Perrin, Purification of Laboratory Chemicals, 3rd édition; Pergamon Press : New York, 1985). Si ce sont des liquides ils sont distillés sous vide et stockés sous atmosphère d'azote.

Les chromatographies sur colonne sont mises en oeuvre avec du gel de silice SDS 60 A. C. (35 - 70 µm). Les chromatographies sur couche mince sont mises en oeuvre en utilisant des plaques de gel de silice MERCK 60F254.

Tous les produits sont caractérisés par leurs spectres RMN, CG/MS et HRMS. Les spectres RMN sont enregistrés à 20°C sur un appareil Bruker AC 400 MHz ou sur un spectromètre DRX-250 travaillant respectivement à 400 MHz pour 1H et à 100 MHz pour 13C. Les déplacements chimiques sont donnés en ppm/TMS pour l'hydrogène 1H et pour {1H} 13C (δ 77,00 pour C). Les motifs des pics de premier ordre sont indiqués comme s (singulet), d (doublet), t (triplet), q (quadruplet). Les signaux complexes qui ne sont pas de premier ordre sont indiqués comme m (multiplet).

Les chromatographies en phase gazeuse et les spectres de masse (GC/MS) sont enregistrés sur un instrument Agilent Technologies 6890 N avec un détecteur de masse Agilent 5973 N (Ei) et une colonne capillaire apolaire HP5-MS 30m x 0,25 mm (phase stationnaire : 5% de film diphenyldimethylpolysiloxane, 0,25 µm). GC/MS protocole : température initiale 45°C ; temps initial 2 min ; pente de la courbe de température : 2°C/min jusqu'à 50°C puis 10°C/min ; température finale 250°C ; temps final : 10mn.

Les spectres IR sont enregistrés sur un appareil Nicolet 210 FT-IR (sous forme de film fin pour les produits liquides et de pastille de KBr ou dans une solution de tétrachlorure de carbone pour les produits solides). Les spectres de masse FAB+ et les HRMS ont été enregistrés sur un spectromètre JEOL JMS-DX300 (3keV, xénon) dans une matrice de m-nitrobenzylalcool.

### Exemple A : Procédé d'hydroxylation de composés aryles iodés ou bromés en présence d'un système catalytique comprenant du iodure de cuivre et du dibenzoylméthane

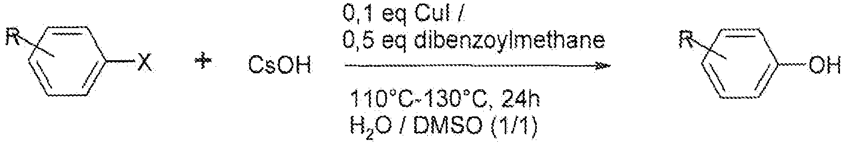

X = I ou Br

### Mode opératoire de l'exemple A :

Après des cycles standard de purge et de remplissage avec de l'azote pur et sec, un tube de Radley (Carrousel station de réaction RR98030") ou un tube de Schlenk séché au four, équipé d'un agitateur magnétique est chargé avec du Cul (0,1 eq.), du CsOH.H2O (3 eq.), de l'halogénophényl si il s'agit d'un solide (1 mmol, 1éq.) et du dibenzoylméthane (0,5 eq.).

Le tube est ensuite purgé puis rerempli avec de l'azote. Si il s'agit d'un liquide l'halogénophényl est additionné sous un courant d'azote avec une seringue à température ambiante, suivi par du DMSO anhydre et dégazé (1,0 mL) et 1 mL d'eau dégazée. Le tube est scellé sous pression positive d'azote, agité et chauffé jusqu'à 130°C (ou à 110°C si cela est précisé) pendant 24 heures. Après refroidissement à la température ambiante, 10 mL de dichlorométhane sont additionnés et 1 mL de HCl (à 37%).

Le mélange est agité pendant deux heures, 130 µL de 1,3-diméthoxybenzène (étalon interne) sont additionnés. Un petit échantillon du mélange réactionnel est prélevé et est filtré à travers un plot de celite®, le solide est ensuite lavé avec du dichlorométhane. Le filtrat est ensuite analysé par chromatographie en phase gazeuse.

Le filtrat est lavé deux fois avec de l'eau. Les phases aqueuses sont rassemblées et extraites avec du dichlorométhane cinq fois. Les phases organiques sont rassemblées, séchées sur sulfate de sodium, filtrées et concentrées sous vide pour donner le produit brut. Le produit brut obtenu est ensuite purifié par chromatographie sur gel de silice avec un mélange d'heptane et d'acétate d'éthyle.

Les produits sont caractérisés par RMN. Leurs rendements en chromatographie en phase gazeuse sont déterminés en corrigeant les facteurs en utilisant des échantillons authentiques des produits attendus.

Pour tester l'étendue de la réaction d'hydroxylation, divers essais ont été effectués à partir de composés aryles iodés ou bromés, porteurs soit de substituants électro-attracteurs soit électro-donneurs.

Les résultats sont présentés dans le tableau 1

**Tableau 1**

| N° exemple | ArX | ArOH | Rendement [%]^{a)} |
|---|---|---|---|
| 1 | Phi | PhOH | 97 |
| 2 | 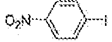 | 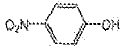 | 22b,90c |
| 3 | 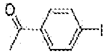 | 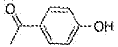 | 90^{c} |
| 4 | 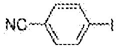 | 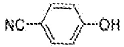 | 91^{c} |
| 5 | 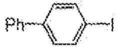 | 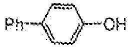 | 95 |
| 6 | 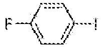 | 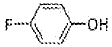 | 70 |
| 7 | 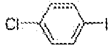 | 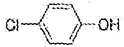 | 75, 95^{d} |
| 8 | 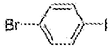 | 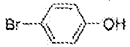 | 84 |
| 9 | 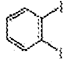 | 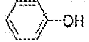 | 95 |
| 10 | 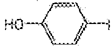 | 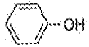 | 95 |
| 11 | 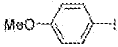 | 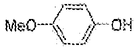 | 90 |
| 12 | 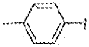 | 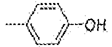 | 82 |
| 13 | 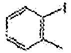 | 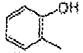 | 84 |
| 14 | 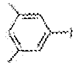 | 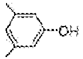 | 96, 71^{c} |
| 15 | 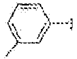 | 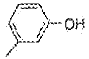 | 84 |
| 16 | 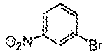 | 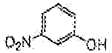 | 78, 7^{c} |
| 17 | 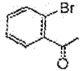 | 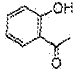 | 83, 17^{b} |
| 18 | 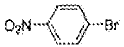 | 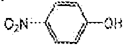 | 82^{c}, 27^{b} |
| 19 | 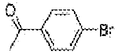 | 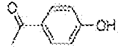 | 84, 20^{b} |
| 20 | 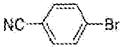 | 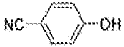 | 94, 24^{b} |

Les rendements correspondent aux rendements isolés
La réaction est effectuée dans les conditions de temps et de température suivantes :
[a] : 24 heures à 130°C
[b] : 24 heures à 130°C en l'absence d'iodure de cuivre
[c] : 24 heures à 110°C
[d] : 36 heures à 130°C

La caractérisation des composés obtenus et les détails des modes opératoires mis en oeuvre sont détaillés ci-après.

### Exemple 1 : Phénol

Suivant le mode opératoire général A, l'iodobenzène (112 µL, 1,0 mmol) est mis à réagir avec de l'hydroxyde de césium pour donner le produit attendu sous forme d'un solide blanc avec un rendement de 97% (éluant : acétate d'éthyle/heptane=20/80).

### Identification

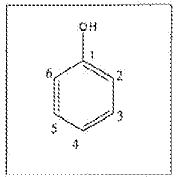

¹H NMR (400 MHz, CDCl₃) : δ 7,15-7,19 (t, 1H, H₄), 7,84-7,88
(t, 2H, N_{3,5}), 6,75-6.77 (d, 2H, H_{2,6}), 4,94 (1H, OH).
¹³C NMR (100 MHz, CDCₗ₃) : δ 155 (C₁), 129,73 (C_{3,5}), 120,88 (C₄), 1115, 33 (C_{2,6}).
GC/MS : rt = 8,96 min, M/Z = 94.
HRMS : 95,0502(M+H). Théorique : 95,0419

### Exemples 2 et 18 : 4-Nitrophénol

Suivant le mode opératoire général A, le 1-iodo-4-nitrobenzène ou le 1-bromo-4- nitrobenzéne (249 mg ou 202 mg, 1,0 mmol) est mis à réagir à 110°C avec de l'hydroxyde de césium pour obtenir le produit attendu sous forme d'un solide jaune à des rendements respectifs de 90% et 82% (acétate d'éthyle/heptane 20/80).

### Identification

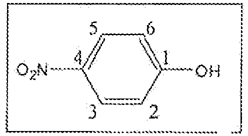

¹H NMR (400 MHz, CDCl₃) : δ 8,12 (m, 2H, H_{3,5}), 6,87 (m, 2H, H_{2,6}), 6,10 (1H, OH).
¹³C NMR (100 MHz, CDCₗ₃): δ 160,31 (C₁), 134,37 (C_{3,5}), 113,51 (C_{2,6}) 102,78 (C₄).
GC/MS : rt = 16,84 min, M/Z = 139.
HRMS : 140,0331 (M+H). Théorique : 140,0348

### Exemples 3 et 19 : 4'-Hydroxyacétophénone

Suivant le mode opératoire général A, la 4'-iodoacétophénone ou la 4'-bromo acétophénone (246 mg ou 199 mg, 1,0 mmol) est mis à réagir avec de l'hydroxyde de césium respectivement à 110 ou 130°C pour donner le produit attendu sous forme d'un solide blanc à des rendements respectifs de 90% et 84% (éluant/acétate d'éthyle/heptane 20/80).

### Identification

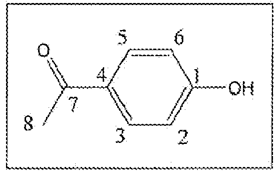

¹H NMR (400 MHz, CDCl₃): δ 6,70-6,71 (m, 4H, H_{2,3}), 4,44 (1H, OH), 3,69 (s, 3H, H₈),
¹³C NMR (100 MHz, CDCl₃): δ 154,06 (C_{1,4}), 149,55 (C₇), 116,09 (C_{3,5}), 114,84 (C_{2,6}), 56,02 (C₈). GC/MS : rt = 15,97 min, M/Z = 136.
HRMS : 135,0452 (M-H). Théorique :135,0446

### Exemples 4 et 20 : 4-Hydroxybenzonitrile

Suivant le mode opératoire général A, le 4-iodobenzonitrile ou le 4-bromobenzonitrile (229 mg ou 182 mg, 1,0 mmol) est mis à réagir respectivement à 110°C ou 130°C avec de l'hydroxyde de césium pour donner le produit attendu sous forme d'un solide blanc à des rendements respectifs de 91% et 94% (éluant : éthyle acétate d'héthyle/heptane = 20/80).

### Identification

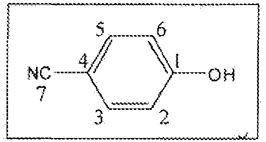

¹H NMR (400 MHz, CDCl₃): δ 7.47-7.50 (m, 2H, H_{3,5}), 6,83-6,86 (m,2H,H_{2,6}), 5,97(1H,NH).
¹³C NMR (100 MHz, CDCl₃) δ 159,79 (C₁), 134,33 (C_{3,5}), 129,39 (C₇), 116,41 (C_{2,6}), 103,71 (C₄). GC/MS **:** rt = 16,13 min, M/Z = 119.
HRMS : 118,0293 (M-H). Théorique : 118,0293

### Exemple 5 : Biphenyl-4-ol

Suivant le mode opératoire général A, le 4-iodobiphényle (280 mg, 1,0 mmol) est mis à réagir avec de l'hydroxyde de césium pour donner le produit attendu sous forme d'un solide brun à un rendement de 95% (éluant acétate d'éthyle/heptane =20/80).

### Identification

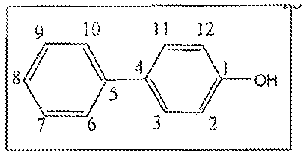

¹H NMR (400 MHz, CDCl₃) :
δ 7,45-7,47 (m, 2H, H_{6,10}), 7,39-7,42 (m, 2H, H_{3,11}), 7,31-7,36 (m, 2H, H_{7,9}), 7,21-7,24 (m, 1H, H₈), 6,83-6,85 (m, 2H, H_{2,12}), 5,10 (1H, OH).
¹³C NMR (100 MHz, CDCl₃) : δ 155,24 (C₁), 140,83 (C₅), 134,00 (C₄), 128,76 (C_{7,9}), 128,41 (C_{3,11}), 126,71 (C_{6,10}), 115,71 (C_{2,12}). GC/MS : rt = 21,03 min, M/Z = 170.
HRMS : 169,0649 (M-H). Théorique : 169,0653.

### Exemple 6 : 4-fluorophénol

Suivant le mode opératoire général A, le 4-fluoroiodobenzène (222 mg, 1,0 mmol) est mis à réagir avec de l'hydroxyde de césium pour donner le produit attendu sous forme d'un solide blanc à un rendement de 70% (éluant acétate d'éthyle/ heptane=20/80).

### Identification

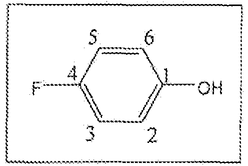

¹H NMR (400 MHz, CDCl₃) : δ 6,83-6,87 (d, 2H, H_{3,5}), 6,68-6,71 (d, 2H, H_{2,6}), 4,66 (1H, OH).
¹³C NMR (100 MHz, CDCl₃) : δ 154,86 (C₁), 132,54 (C_{3,5}), 117,25 (C_{2,6}), 113,15 (C₄).
GC/MS : rt = 12,86 min, M/Z = 112,
HRMS : 113,0403 (M+H). Théorique : 113, 0403

### Exemple 7 : 4-Chlorophénol

Suivant le mode opératoire généra! A, le 4-chloroidobenzène (250 mg, 1,0 mmol) est mis à réagir avec de l'hydroxyde de césium pour donner le produit attendu sous forme d'un solide blanc à un rendement de 75% (éluant acétate d'éthyle/heptane =10/90).

### Identification

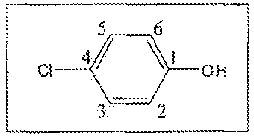

¹H NMR (400 MHz, CDCl₃) : δ 7,07-7,09 (d, 2H, H_{3,5}), 6,65-6,68 (d, 2H, H_{2,6}), 5,41 (1H, OH).
¹³C NMR (100 MHz, CDCl₃) : δ 154,14 (C₁), 129,64 (C_{3,5}), 125,9 (C₄). 116,46 (C_{2,6}).
GC/MS : rt = 13,09 min, M/Z = 128.
HRMS : 129, 0128 (M+H). Théorique : 129,0107

### Exemple 8 : 4-Bromophénol

Suivant le mode opératoire général A, le 4-iodobromobenzène (282 mg, 1,0 mmol) est mis à réagir avec de l'hydroxyde de césium pour donner le produit attendu sous forme d'un solide brun à un rendement de 84% (éluant acétate d'éthyle/heptane=20/80).

### Identification

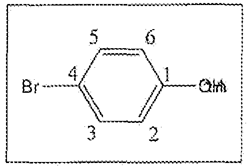

¹H NMR (400 MHz, CDCl₃): δ 7,23-7,26 (d, 2H, H_{3,5}), 6,63-6,65 (d, 2H, H_{2,6}), 5,05 (1H, OH).
¹³C NMR (100 MHz, CDCl₃) : δ 154,86 (C₁), 132,54 (C_{3,5}), 117,25 (C_{2,6}), 113,15 (C₄).
GC/MS : rt = 14,06 min, M/Z = 172.
HRMS : 170,9446 (M-H). Théorique : 170,9446

### Exemple 11 : 4-méthoxyphenol

Suivant le mode opératoire général A, le 4-méthoxyiodobenzène (246 mg, 1,0 mmol) est mis à réagir avec de l'hydroxyde de césium pour donner le produit attendu sous forme d'un solide blanc à un rendement de 90% (éluant : acétate d'éthyle /heptane=20/80),

### Identification

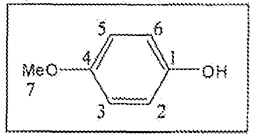

¹H NMR (400 MHz, CDCl₃): δ 6,68-6,69 (m, 3H, H_{2,3,5,6}), 5,38 (1H, OH), 3,67 (s, 3H, H₇).
¹³C NMR (100 MHz, CDCl₃) : δ 153,80 (C₁), 149,68 (C₁), 116,13 (C_{3,5}), 114, 95 (C_{2,6}, 55,89 (C₇),
GC/MS : rt = 12,71 min, M/Z = 124.
HRMS : 125,0617 (M+H). Théorique : 125,0603

### Exemple 12 : p-Cresol

Suivant le mode général A, le 4-iodotoluène (218 mg, 1,0 mmol) est mis à réagir avec de l'hydroxyde de césium pour donner le produit attendu sous forme d'un solide blanc à un rendement de 82% (éluant **:** éthylo/acétate/heptane=20/80).

### Identification

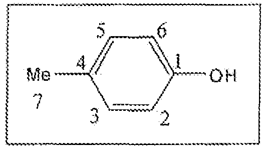

¹H NMR (400 MHz, CDCl₃): δ 6,89-6,91 (d, 2H, H_{3,5}), 6,61-6,63 (d, 2H, H_{2,6}), 5,86 (1H, OH), 2,15 (s, 3H, H₇). ¹³C NMR (100 MHz, CDCl₃) : δ 153,15 (C₁), 130,08 (C₄), 130,08 (C_{3,5}), 115,08 (C_{2,6}), 20,04 (C₇).
GC/MS : rt = 10.56 min, M/Z = 108.
HRMS : 109,0668 (M+H). Théorique : 109,0653

### Exemple 13 : o-Cresol

Suivant le mode général A, le 2-méthyliodobenzène (128 µL, 1,0 mmol) est mis à réagir avec l'hydroxyde de césium pour donner le produit attendu sous forme d'un solide blanc à un rendement de 84% (éluant : acétate d'éthyle /heptane=20/80).

### Identification

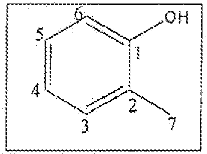

¹H NMR (400 MHz, CDCl₃) : δ 6,95-7,03 (m, 2H, H_{4,5}), 6,73-6,77 (m, 1H, H₃), 6,64-6,67 (m, 1H. H₆), 5,01 (1H, OH), 2,15 (s, 3H, H₇).
¹³C NMR (100 MHz, CDCl₃) : δ 153,93 (C₁), 131,22 (C₃), 127,23 (C₅), 124,04 (C₂), 120,98 (C₄), 115,27 (C₆), 15,90 (C₇).
GC/MS : rt = 10,68 min, M/Z = 108.
HRMS : 107,0499 (M-H). Théorique : 107,0497

### Exemple 14 : 3,5-dimethylphénol

Suivant le mode général A, le 3,5-diméthyliodobenzène (145 µL, 1,0 mmol) est mis à réagir avec l'hydroxyde de césium pour donner le produit attendu sous forme d'un solide blanc à un rendement de 96% (éluant : dichlorométhane/heptane = 20/80).

### Identification

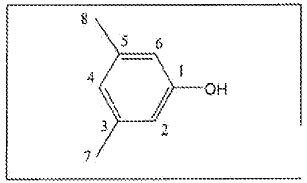

¹H NMR (400 MHz, CDCl₃) : δ 6,50 (s, 1H, H₄), 6,38 (s, 2H, H_{2,6}), 4,67 (1H, OH), 2,17 (d, 6H, H_{7,8}). ¹³C NMR (100 MHz, CDCl₃) : δ 155,53 (C₁),
139,72 (C_{3,5}), 122,71 (C₄), 113,15 (C_{2,6}), 21,48 (C_{7,8}). GC/MS : rt = 12,21 min, M/Z = 122.
HRMS : 123,0816 (M+H). Théorique : 123,0810

### Exemple 15 : m-Cresol

Suivant le mode opératoire général A, le 3-méthyliodobenzène (128 µL, 1,0 mmol) est mis à réagir avec l'hydroxyde de césium pour donner le produit attendu sous forme d'un solide blanc à un rendement de 84% (éluant : acétate d'éthyle /heptane=20/80).

### Identification

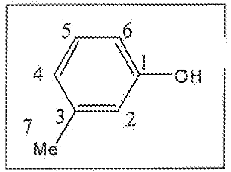

¹H NMR (400 MHz, CDCl₃) : δ 7,52-7,54 (d, 2H, H_{4,6}), 6,69 (s, 1H, H₂), 6,34-6,36 (m, 1H, H₅), 4,89 (1H, OH), 2,29 (s, 3H, H₇).
¹³C NMR (100 MHz, CDCl₃) : δ 156,33 (C₁), 142,64 (C₃), 139,59 (C₅), 117,40 (C₄), 115.14 (C_{2,6}), 28,12 (C₇). GC/MS : rt = 10,88 min, M/Z = 108.
HRMS : 107,0498 (M-H). Théorique : 107,0409

### Exemple 16 : 3-Nitrophénol

Suivant le mode opératoire général A, le 1-bromo-3-nitrobenzène (202 mg, 1,0 mmol) est mis à réagir avec l'hydroxyde de césium pour donner le produit attendu sous forme d'un solide rouge à un rendement de 78% (éluant : acétate d'éthyle /heptane=20/80).

### Identification

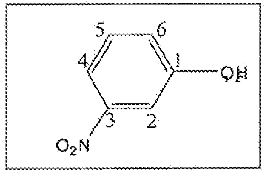

¹H NMR (400 MHz, CDCl₃): δ 7,72-7.74 (m, 1H, H₅), 7,64-7,65 (m, 1H, H₄), 7,31-7,35 (t, 1H, H₆), 7,12-7,14 (m, 1H, H₂), 5,40 (1H, OH).
¹³C NMR (100 MHz, CDCl₃) : δ 156,33 (C₁), 149,15 (C₃), 130,42(C₅), 122,18(C₆), 115,93(C₄) 110,62 (C₂).
GC/MS : rt = 16,35 min, M/Z = 139.
HRMS : 138,0194 (M-H). Théorique : 138,0191

### Exemple 17 : 2'-Hydroxyacétophénone

Suivant le mode opératoire général A, la 2'-bromoacétophénone (199 mg, 1,0 mmol) est mise à réagir avec l'hydroxyde de césium pour donner le produit attendu sous forme d'une huile à un rendement de 83% (éluant : acétate d'éthyle /heptane=20/80).

### Identification

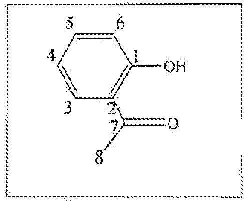

¹H NMR (400 MHz, CDCl₃), δ 12,19 (s, 1H, OH), 7,64-7,68 (m, 1H, H₅), 7,31-7,41 (m, 1H, H₃), 6,88-6,90 (m, 1H, H₄), 6,80~6,84(m, 1H, H₆), 2,55(s, 3H, H₈),
¹³C NMR (100 MHz, CDCl₃) : δ 204,82 (C₇), 162,44 (C₁), 136,66 (C₅), 130,69 (C₃), 119,66 (C₂), 118,99 (C₄), 118,60 (C₆), 26,93 (C₈).
GC/MS : rt = 15,32 min, M/Z = 136.
HRMS : 135,0444 (M-H). Théorique : 135,0446

### Exemple B : procédé d'hydroxylation de composés aryles bromés en présence de iodure de sodium et d'un système catalytique comprenant du iodure de cuivre et la N,N'-diméthyléthylenediamine ;

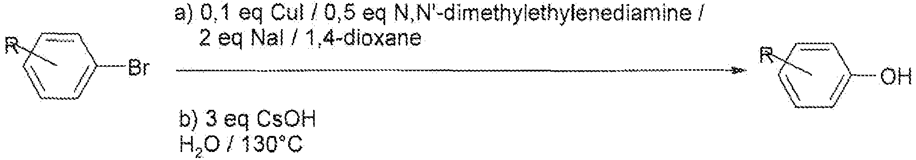

### Mode opératoire de l'exemple B :

Après des cycles standards de purge et de remplissage avec de l'azote pur et sec, un tube de Radley (Carrousel « station de réaction RR98030") ou un tube de Schlenk, séché au four équipé avec un agitateur magnétique est chargé avec du Cul (0,1 eq.), du Nal (2 eq) et le bromure de phényl, si il s'agit d'un solide (1 mmol, 1 eq). Le tube est ensuite purgé, puis rerempli avec de l'azote. Si il s'agit d'un liquide le bromure de phényl est additionné sous un flux d'azote avec une seringue à température ambiante, suivi par de la DMEDA NN'dimethyléthylene diamine (0,5 eq.) et du 1,4-dioxane (1,0 mL) dégazé. Le tube est scellé sous pression positive d'azote, agité et chauffé jusqu'à 110 °C. Après 6h de réaction, est additionné sous flux d'azote, le CsOH.H2O (3 eq.), et 1 mL d'eau dégazée. Le tube est scellé sous pression positive d'azote, agité et chauffé jusqu'à 130 °C pendant 24 heures.

Après refroidissement à température ambiante, 10 mL de dichlorométhane sont additionnés et 1 mL de HCl (à 37%). Le mélange est agité pendant deux heures, 130 µL de 1,3-diméthoxybenzène (étalon interne) sont additionnés. Un petit échantillon du milieu réactionnel est prélevé et est filtré sur un plot de célite®, le solide est ensuite lavé avec du dichlorométhane. Le filtrat est analysé par chromatographie en phase gazeuse.

Le filtrat est lavé deux fois avec de l'eau, les phases aqueuses réunies sont extraites avec du dichlorométhane cinq fois. Les phases organiques sont réunies et séchées, sur sulfate de sodium, filtrées et concentrées sous vide pour donner le produit brut qui est purifié par chromatographie sur gel de silice avec un mélange d'heptane et d'acétate d'éthyle. Les produits sont caractérisés par RMN. Les rendements par chromatographie gazeuse sont déterminés en effectuant une correction des facteurs en utilisant des échantillons authentiques des produits attendus.

**Tableau 2**

| N°exemple | ArBr | ArOH | Rendement [%] |
|---|---|---|---|
| 21 | 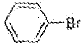 | 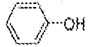 | 85, 75^{a} |
| 22 | 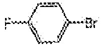 | 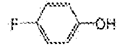 | 70 |
| 23 | 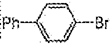 | 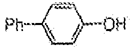 | 90 |
| 24 | 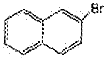 | 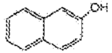 | 88 |
| 25 | 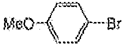 | 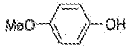 | 87 |
| 26 | 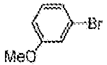 | 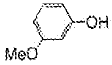 | 85 |
| 27 | 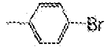 | 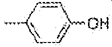 | 80, 83^{b} |

Les rendements sont les rendements isolés
[a] : le 1,4-dioxane est substitué par le DMSO
[b] : le 1,4-dioxane est substitué par le toluène

### Exemple 23 : Biphényl-4-ol

Suivant le mode opératoire général B, le 4-bromobiphényl (232 mg, 1,0 mmol) est mis à réagir avec de l'hydroxyde de césium pour donner le produit attendu sous forme d'un solide brun à un rendement de 90% (éluant : acétate d'éthyle/heptane=20/80).

### Identification

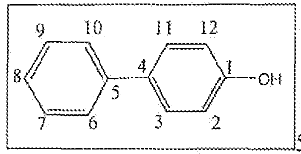

¹H NMR (400 MHz, CDCl₃) :
δ 7,45-7,47 (m, 2H, H_{6,10}), 7,39-7,42 (m, 2H, H_{3,11}), 7,31-7,36 (m, 2H, H_{7,9}), 7,21-7,24 (m, 1H, H₈), 6,83-6,85 (m, 2H, H_{2,12}), 5,10 (1H, OH).
¹³C NMR (100 MHz, CDCl₃) : δ 155,24 (C₁), 140,83 (C₅), 134,00 (C₄), 128,76 (C_{7,9}), 128,41 (C_{3,11}), 126,71 (C_{6,10}), 115,71 (C_{2,12}).
GC/MS: rt = 21,03 min, M/Z = 170 .
HRMS : 169,0649 (M-H). Théorique : 169,0653.

### Exemple 24: 2-naphtol

Suivant le mode opératoire général B, le 2-bromonaphtalène (207 mg, 1,0 mmol) est mis à réagir avec de l'hydroxyde de césium pour donner le produit attendu sous forme d'un solide blanc à un rendement de 88% (éluant : acétate d'éthyle /heptane=20/80).

### Identification

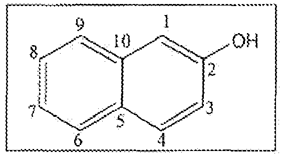

¹H NMR (400 MHz, CDCl₃) : δ 7,65-7,69 (m, 2H, H₄₆), 7,60-7,58 (m, 1H, H₉), 7,32-7,36 (m, 1H, H₈), 7,22-7,26 (m, 1H, H₇), 7,05-7,06 (m, 1H, H₁), 7,00-7,03 (m, 1H, H₃), 5,06 (1H, OH).
¹³C NMR (100 MHz, CDCl₃) : δ 153,61 (C₂), 134,75 (C₁₀), 129,96 (C₄), 128,93 (C₅), 128,01 (C₆), 126,76 (C8), 126,41 (C9), 123,67 (C7), 117,73 (C3), 109,61 (C1).
GC/MS : rt = 13,95 min, M/Z = 144.
HRMS : 143,0495 (M-H). Théorique : 143,0497

### Exemple 26 : m-méthoxyphénol

Suivant le mode opératoire général B, le 3-méthoxybromobenzène (127 µL. 1,0 mmol) est mis à réagir avec de l'hydroxyde de césium pour donner le produit attendu sous forme d'un huile à un rendement de 85% (éluant : acétate d'éthyle /heptane=10/90).

### Identification

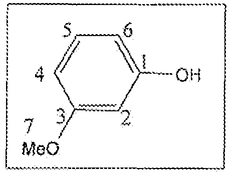

¹H NMR (400 MHz, CDCl₃) : δ 7,03-7,08 (m, 1H, H₅), 6,41-643 (m, 1H, H₄). 6,34-6,36 (m, 2H, H_{2,6}), 5,06 (1H, OH), 3,70 (s, 3H, H₇).
¹³C NMR (100 MHz, CDCl₃) : δ 161,11 (C₃), 156,59 (C₁), 30,18 (C₅), 106,44 (C₄), 101,54 (C₂), 55,31 (C₇). GC/MS : rt = 11,63 min, M/Z= 124.
HRMS : 123,0456 (M-H). Théorique : 123,0446

### Exemple C : influence du ligand

Pour tester l'étendue de la réaction d'hydroxylation, divers essais ont été effectués à partir de iodobénzene, en faisant varier la nature du ligand

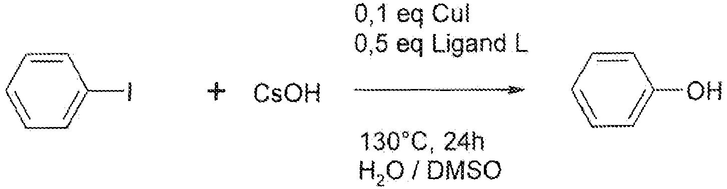

### Mode opératoire de l'exemple C :

Après des cycles standard de purge et de remplissage avec de l'azote pur et sec, un tube de Radley (Carrousel station de réaction RR98030") ou un tube de Schlenk séché au four, équipé d'un agitateur magnétique est chargé avec du Cul (0,1 eq.), du CsOH.H2O (3 eq.), de iodobenzéne si il s'agit d'un solide (1 mmol, 1éq.) et le ligand L (0,5 eq.) si il s'agit d'un solide. Le tube est ensuite purgé puis rerempli avec de l'azote. Si il s'agit d'un liquide le iodobenzéne est additionné sous un courant d'azote avec une seringue à température ambiante et le ligand L (0,5 eq.) si il s'agit d'un liquide est additionné avec une seringue, suivi par du DMSO anhydre et dégazé (1,0 mL) et 1 mL d'eau dégazée. Le tube est scellé sous pression positive d'azote, agité et chauffé jusqu'à 130°C pendant 24 heures. Après refroidissement à la température ambiante, 10 mL de dichlorométhane sont additionnés et 1 mL de HCl (à 37%). Le mélange est agité pendant deux heures, 130 µL de 1,3-diméthoxybenzène (étalon interne) sont additionnés. Un petit échantillon du mélange réactionnel est prélevé et est filtré à travers un plot de celite®, le solide est ensuite lavé avec du dichlorométhane. Le filtrat est ensuite analysé par chromatographie en phase gazeuze.

Le filtrat est lavé deux fois avec de l'eau. Les phases aqueuses sont rassemblées et extraites avec du dichlorométhane cinq fois. Les phases organiques sont rassemblées, séchées sur sulfate de sodium, filtrées et concentrées sous vide pour donner le produit brut.

Les rendements en chromatographie en phase gazeuse sont déterminés en corrigeant les facteurs en utilisant des échantillons authentiques des produits attendus.

Les résultats sont présentés dans le tableau 3.

**Tableau 3**

| N°exemple | Ligands L | Rendement (%) |
|---|---|---|
| L1 | 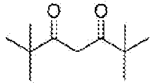 | 95 |
| L2 | 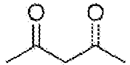 | 30 |
| L3 | 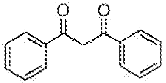 | 97 |
| L4 | 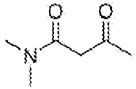 | |
| L5 | 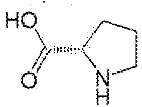 | 70 |
| L6 | 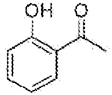 | 85 |
| L7 | 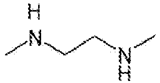 | 84 |
| L8 | 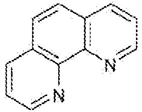 | 75 |

Le rendement est déterminé avec le 1,3-dimethoxybenzène comme standard.

### Exemple D: synthèse de phénol et de diphénylether par hydroxylation de l'iodobenzéne en présence d'un système catalytique comprenant du iodure de cuivre et de la 1,3-ditertiobutyl-propane-1,3-dione.

### Mode opératoire de l'exemple D :

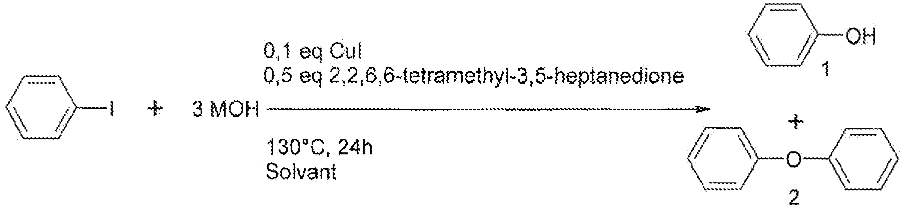

Après des cycles standard de purge et de remplissage avec de l'azote pur et sec, un tube de Radley (Carrousel station de réaction RR98030") ou un tube de Schlenk séché au four, équipé d'un agitateur magnétique est chargé avec du Cul (0,1 eq.), du CsOH.H2O (3 eq.), de l'iodobenzéne si il s'agit d'un solide (1 mmol, 1éq.). Le tube est ensuite purgé puis rerempli avec de l'azote. Si il s'agit d'un liquide l'halogénophényl est additionné sous un courant d'azote avec une seringue à température ambiante et la 2,2,6,6-tetraméthyl-3,5heptanedione (0,5 eq.), suivi par du solvant anhydre et dégazé (2,0 mL) s'il s'agit d'un co-solvant le volume totale est de 2 mL. Le tube est scellé sous pression positive d'azote, agité et chauffé jusqu'à 130° pendant 24 heures. Après refroidissement à la température ambiante, 10 mL de dichlorométhane sont additionnés et 1 mL de HCl (à 37%).

Le mélange est agité pendant deux heures. 130 µL de 1,3-diméthoxybenzène (étalon interne) sont additionnés. Un petit échantillon du mélange réactionnel est prélevé et est filtré à travers un plot de celite®, le solide est ensuite lavé avec du dichlorométhane. Le filtrat est ensuite analysé par chromatographie en phase gazeuze.

Les rendements en chromatographie en phase gazeuse sont déterminés en corrigeant les facteurs en utilisant des échantillons authentiques des produits attendus.

### Exemple E : Divers essais ont été effectués à partir de iodobenzéne, en faisant varier la nature du métal M ainsi que la nature du solvant

Les résultats sont présentés dans le tableau 4

**Tableau 4**

| N°exemple | Solvant | MOH | Rendement (%) | |
|---|---|---|---|---|
| | | | 1 | 2 |
| S1 | NMP | KOH | 20 | 20 |
| S2 | H₂O | KOH | 20 | 0 |
| S3 | DMSO | KOH | 50 | 20 |
| S4 | DMSO/H₂O 7/1 | KOH | 19 | 28 |
| S5 | DMF/H₂O 7/1 | KOH | 15 | 20 |
| S6 | DMSO/H₂O 3/1 | KOH | 60 | 20 |
| S7 | DMF/H₂O 3/1 | KOH | 70 | 15 |
| S8 | DMSO/H₂O 3/1 | CsOH | 80 | 5 |
| S9 | MIBK/H₂O 3/1 | CsOH | 30 | 20 |
| S10 | NMP/H₂O 3/1 | CsOH | 30 | 20 |
| S11 | DMSO/H₂O 1/1 | CsOH | 95 | 0 |
| S12^{[b]} | DMSO/H₂O 1/1 | CsOH | 45 | 0 |
| S13^{[c]} | OMSO/H₂O 1/1 | CsOH | 40 | 25 |
| S14 | DMSO/H₂O 1/1 | KOH | 70 | 0 |

Le rendement est déterminé avec le 1,3-diméthoxybenzène comme standard.
[b] : réaction effectuée avec 1,5 eq de CsOH
[c] : réaction effectuée à 120°C

## Revendications

1. Procédé d'hydroxylation de composés aryles halogénés comprenant une réaction d'hydroxylation mise en oeuvre à une température inférieure à 150°C, en présence d'un système catalytique consistant en un catalyseur à base de cuivre et un ligand L selon le schéma réactionnel suivant : 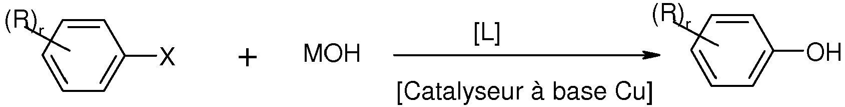
◆ R est choisi parmi les groupes à effet inductif accepteur choisi dans le groupe constitué par le groupe NO₂, les esters, le groupe CN, un atome d'halogène, un groupe alkoxy et les groupes à effet mésomère donneur choisi dans le groupe constitué par le groupe phényle, le groupe hydroxy (OH), un alkyle en C1 à C10, de préférence en C1 à C6, linéaire ou ramifié, un atome d'halogène, un atome d'hydrogène, un groupe alkoxy, ou R forme avec le phényle un dérivé naphtyl;
◆ r étant compris entre 0 et 5 ;
◆ X étant un atome d'halogène ;
◆ M étant choisi parmi les cations alcalins ou alcalino-terreux ;
◆ L représente un composé de formule (I) 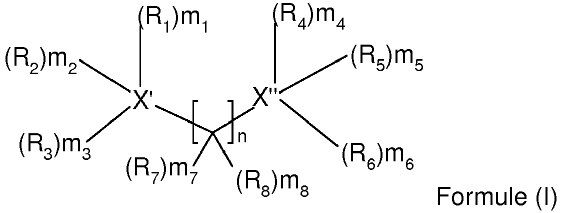 dans laquelle
◆ X', X", identiques ou différents sont choisis parmi l'atome d'azote, le groupe C=O, le groupe C=S et le groupe C-OH ;
◆ m₁ à m₈ identiques ou différents représentent 0 ou 1 ;
◆ n représente 1 ou 2 ;
◆ R₁, R₂, R₃, R₄, R₅, R₆, R₇ et R₈ identiques ou différents sont choisis parmi :
- un atome d'hydrogène ;
- un groupe hydrocarboné ramifié, linéaire, monocyclique ou polycyclique, comportant de 1 à 20 atomes de carbone, et pouvant comporter une ou plusieurs insaturations sous forme de double(s) et/ou triple(s) liaison(s), de préférence méthyle, isobutyle, phényle ;
- une amine primaire, secondaire ou tertiaire -NR'R", avec R' et R" identiques ou différents représentant un groupe alkyle en C₁ à C₁₀, de préférence en C₁ à C₆, linéaire ou ramifié, de préférence méthyle ;
- un groupe hydroxyle ;
◆ ou R₄, X" et R₇ forment un groupe hétérocyclique, de préférence pyrrolidine ;
◆ ou R₁, X' et R₇ forment un groupe phénol ;
◆ ou R₁, X', X", R₇ et R₄ forment un groupe polycyclique constitué par au moins 3 cycles saturés et/ou insaturés ou par au moins 3 cycles dont l'un seul d'entre eux ou deux d'entre eux est (sont) aromatique(s) et formant entre eux des systèmes ortho- ou ortho- et peri-condensés, de préférence le groupe phénanthroline.

2. Procédé selon la revendication 1 pour lequel dans la formule (I):
◆ R₁, R₂, R₃, R₄, R₅, R₆, R₇ et R₈ identiques ou différents représentent :
- un atome d'hydrogène ;
- un groupe alkyle, en C₁ à C₁₀;
- un groupe aryle; ou
- une amine primaire, secondaire ou tertiaire -NR'R", avec R' et R", identiques ou différents, représentant un atome d'hydrogène, un groupe alkyle en C₁ à C₁₀;
- un groupe hydroxyle ;
◆ R₇ et R₈ identiques ou différents représentent un atome d'hydrogène ; ou
◆ R₄, X" et R₇ forment un groupe hétérocyclique; ou
◆ R₁, X' et R₇ forment un groupe phénol ; ou
◆ R₁, X', X", R₇ et R₄ forment un groupe polycyclique constitué par au moins 3 cycles saturés et/ou insaturés ou par au moins 3 cycles dont l'un seul d'entre eux ou deux d'entre eux est (sont) aromatique(s) et formant entre eux des systèmes ortho- ou ortho- et peri-condensés.

3. Procédé selon l'une quelconque des revendications précédentes pour lequel dans la formule (I) :
◆ X', X", identiques ou différents sont choisis parmi l'atome d'azote, le groupe C=O, le groupe C=S et le groupe C-OH ;
◆ n représente 1 ;
◆ m₄, m₇ et m₈ représentent 1 ; m₁, m₂, m₃, m₅ et m₆ représentent 0 ou 1 ;
◆ R₁, R₂, R₃, R₅, et R₆ identiques ou différents sont choisis parmi :
- un atome d'hydrogène ;
- un groupe hydrocarboné ramifié, linéaire ou cyclique (mono- ou polycyclique), comportant de 1 à 20 atomes de carbone, et pouvant comporter une ou plusieurs insaturations sous forme de double(s) et/ou triple(s) liaison(s), de préférence méthyle, isobutyle, phényle ;
- une amine primaire, secondaire ou tertiaire -NR'R", avec R' et R" identiques ou différents représentant un groupe alkyle en C₁ à C₁₀, de préférence en C₁ à C₆, linéaire ou ramifié, de préférence méthyle ;
- un groupe hydroxyle ;
◆ R₈ représente H et R₄, X" et R₇ forment un groupe hétérocyclique, de préférence pyrrolidine ; ou
◆ X', X", identiques ou différents sont choisis parmi l'atome d'azote, le groupe C=O, le groupe C=S et le groupe C-OH ;
◆ n représente 1 ;
◆ m₂ à m₆ représentent 0 ou 1 ; m₁, m₇ et m₈ représentent 1 ;
◆ R₁ à R₆, identiques ou différents, sont choisis parmi :
- un atome d'hydrogène ;
- un groupe hydrocarboné ramifié, linéaire ou cyclique (mono- ou polycyclique), comportant de 1 à 20 atomes de carbone, et pouvant comporter une ou plusieurs insaturations sous forme de double(s) et/ou triple(s) liaison(s), de préférence méthyle, isobutyle, phényle ;
- une amine primaire, secondaire ou tertiaire -NR'R", avec R' et R" identiques ou différents représentant un groupe alkyle en C₁ à C₁₀, de préférence en C₁ à C₆, linéaire ou ramifié, de préférence méthyle ;
- un groupe hydroxyle ;
◆ R₁, X' et R₇ forment un groupe phénol et R₈ représente H; ou
◆ X', X", identiques ou différents sont choisis parmi l'atome d'azote, le groupe C=O, le groupe C=S et le groupe C-OH ;
◆ n représente 2 ;
◆ m₂, m₃ m₅, m₆ et m₈ représentent 0 ou 1 ; m₁, m₄ et m₇ représentent 1 ;
◆ R₂, R₃, R₅, R₆ et R₈, identiques ou différents, sont choisis parmi :
- un atome d'hydrogène ;
- un groupe hydrocarboné ramifié, linéaire ou cyclique (mono- ou polycyclique), comportant de 1 à 20 atomes de carbone, et pouvant comporter une ou plusieurs insaturations sous forme de double(s) et/ou triple(s) liaison(s), de préférence méthyle, isobutyle, phényle ;
- une amine primaire, secondaire ou tertiaire -NR'R", avec R' et R" identiques ou différents représente un groupe alkyle en C₁ à C₁₀, de préférence en C₁ à C₆, linéaire ou ramifié, de préférence méthyle ;
- un groupe hydroxyle ;
◆ R₁, X', X", R₇ et R₄ forment un groupe polycyclique comprenant au moins 3 cycles saturés et/ou insaturés ou au moins 3 cycles dont l'un ou deux d'entre eux sont aromatiques et forment entre eux des systèmes ortho- ou ortho- et peri-condensés, de préférence le groupe phénanthroline.

4. Procédé selon l'une quelconque des revendications précédentes pour lequel L est choisi parmi

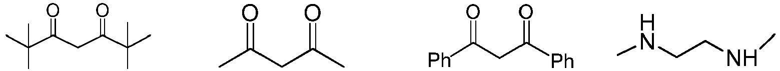

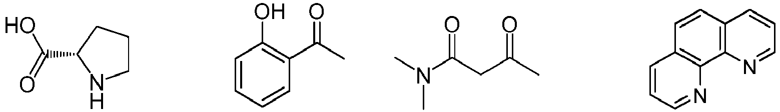

5. Procédé selon l'une quelconque des revendications 1 à 4 pour lequel X est choisi parmi le brome ou l'iode.

6. Procédé selon l'une quelconque des revendications 1 à 5 pour lequel R est un groupe à effet inductif accepteur choisi parmi le groupe NO₂, le groupe CO₂Me, le groupe CN, un atome d'halogène et le groupe méthoxy.

7. Procédé selon l'une quelconque des revendications 1 et 5 pour lequel X est le brome et R est un groupe à effet mésomère donneur choisi dans le groupe constitué par phényle, un groupe hydroxy, un groupe méthyle, l'atome de fluor, l'atome d'hydrogène et un groupe méthoxy ou R forme avec le phényl un groupe naphtyl.

8. Procédé selon l'une quelconque des revendications précédentes pour lequel le cation M est choisi parmi potassium ou césium.

9. Procédé selon l'une quelconque des revendications précédentes **caractérisé en ce que** la réaction d'hydroxylation est mise en oeuvre en deux étapes selon le schéma réactionnel suivant : 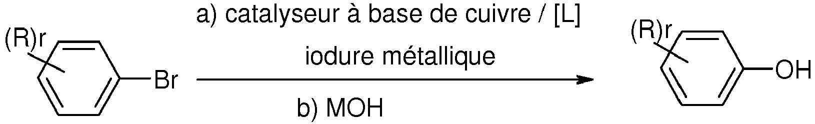 avec
R étant défini aux revendications 1, 6 ou 7 ;
r étant défini à la revendication 1 ;
L étant défini aux revendications 1 à 4 ; et
M étant défini aux revendications 1 et 8 ;
la première étape (a) correspondant à une substitution nucléophile du bromobenzène par un iodure métallique en présence d'un catalyseur à base de cuivre et d'un ligand,
la deuxième étape correspondant à la réaction d'hydroxylation en présence d'un catalyseur à base de cuivre et d'un ligand.

10. Procédé selon la revendication 9 pour lequel l'iodure métallique est choisi parmi NaI, KI ou CsI.

11. Procédé selon l'une quelconque des revendications 9 ou 10 pour lequel le ratio molaire entre le nombre de moles d'iodure métallique et le nombre de moles de composé bromé varie entre 0,1 et 4, de préférence entre 1 et 3.

12. Procédé selon l'une quelconque des revendications précédentes pour lequel les catalyseurs à base de cuivre sont choisis parmie cuivre métallique les oxydes de cuivre (I) ou cuivre (II), les hydroxydes de cuivre (I) ou cuivre (II), les sels inorganiques ou organiques de cuivre (I) ou cuivre (II) et les complexes de cuivre (I) ou cuivre (II) avec des ligands usuels.

13. Procédé selon l'une quelconque des revendications précédentes pour lequel le ratio molaire entre le nombre de moles de catalyseur à base de cuivre et le nombre de moles de composé arylé halogéné est compris entre 0,001 et 0,5.

14. Procédé selon l'une quelconque des revendications précédentes pour lequel le ratio molaire entre le nombre de moles de ligand L et le nombre de moles de composé arylé halogéné varie entre 0,001 et 0,9.

15. Procédé selon l'une quelconque des revendications précédentes pour lequel le ratio molaire entre le nombre de moles de MOH et le nombre de moles de composé arylé bromé varie entre 0,1 et 5.

## Patentansprüche

1. Verfahren zur Hydroxylierung von halogenierten Arylverbindungen, das eine Hydroxylierungsreaktion umfasst, die bei einer Temperatur kleiner als 150 °C bei Anwesenheit eines katalytischen Systems durchgeführt wird, das aus einem Katalysator auf Basis von Kupfer und einem Liganden L besteht, gemäß dem folgenden Reaktionsschema: 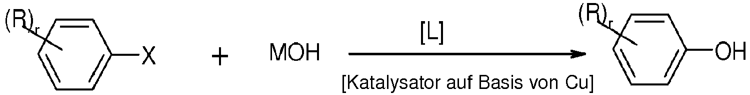
• wobei R ausgewählt ist aus Gruppen mit induktiver Akzeptorwirkung, ausgewählt aus der Gruppe bestehend aus der NO₂-Gruppe, Estern, der CN-Gruppe, einem Halogenatom, einer Alkoxy-Gruppe und Gruppen mit mesomerer Donatorwirkung, ausgewählt aus der Gruppe bestehend aus der Phenyl-Gruppe, der Hydroxy-Gruppe (OH), einem C1- bis C10-Alkyl, vorzugsweise C1-bis C6-Alkyl, linear oder verzweigt, einem Halogenatom, einem Wasserstoffatom, einer Alkoxy-Gruppe, wobei R mit dem Phenyl ein Naphthyl-Derivat bildet;
• wobei r zwischen 0 und 5 liegt;
• wobei X ein Halogenatom ist;
• wobei M ausgewählt ist aus den Alkalikationen oder Erdalkalikationen;
• wobei L eine Verbindung der Formel (I) darstellt, 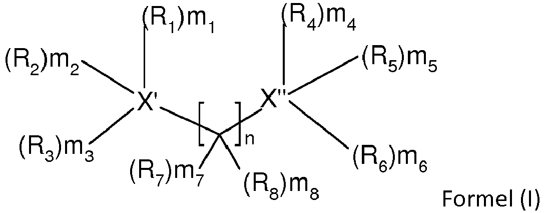 in der
• X', X", identisch oder unterschiedlich, ausgewählt sind aus dem Stickstoffatom der C=O-Gruppe, der C=S-Gruppe und der C-OH-Gruppe;
• m₁ bis m₈, identisch oder unterschiedlich, 0 oder 1 darstellen;
• n 1 oder 2 darstellt;
• R₁, R₂, R₃, R₄, R₅, R₆, R₇ und R₈, identisch oder unterschiedlich, ausgewählt sind aus:
- einem Wasserstoffatom;
- einer verzweigten, linearen, monozyklischen oder polyzyklischen Kohlenwasserstoffgruppe, die 1 bis 20 Kohlenstoffatome aufweist und eine oder mehrere Ungesättigtheiten in Form von Doppel- und/oder Dreifachbindung(en), vorzugsweise Methyl, Isobutyl, Phenyl, aufweisen kann;
- einem primären, sekundären oder tertiären Amin -NR'R", wobei R' und R", identisch oder unterschiedlich, eine C₁- bis C₁₀- Alkylgruppe, vorzugsweise eine C₁- bis C₆-Alkylgruppe, linear oder verzweigt, vorzugsweise Methyl, darstellen;
- einer Hydroxylgruppe;
• oder R₄, X" und R₇ eine heterozyklische Gruppe, vorzugsweise Pyrrolidin bilden;
• oder R₁, X' und R₇ eine Phenolgruppe bilden;
• oder R₁, X', X", R₇ und R₄ eine polyzyklische Gruppe bilden, die aus mindestens drei gesättigten und/oder ungesättigten Zyklen oder aus mindestens drei Zyklen, von denen ein einziger unter ihnen oder zwei unter ihnen aromatisiert ist (sind) und untereinander ortho- oder ortho- und peri-kondensierte Systeme bilden, vorzugsweise die Phenanthrolin-Gruppe, gebildet ist.

2. Verfahren nach Anspruch 1, für das in der Formel (I):
• R₁, R₂, R₃, R₄, R₅, R₆, R₇ und R₈, identisch oder unterschiedlich, darstellen:
- ein Wasserstoffatom;
- eine C₁- bis C₁₀-Alkylgruppe;
- eine Arylgruppe; oder
- ein primäres, sekundäres oder tertiäres Amin -NR'R", wobei R' und R", identisch oder unterschiedlich, ein Wasserstoffatom, eine C₁- bis C₁₀-Alkylgruppe darstellen;
- eine Hydroxylgruppe;
• R₇ und R₈, identisch oder unterschiedlich, ein Wasserstoffatom darstellen; oder
• R₄, X" und R₇ eine heterozyklische Gruppe bilden; oder
• R₁, X' und R₇ eine Phenolgruppe bilden; oder
• R₁, X', X", R₇ und R₄ eine polyzyklische Gruppe bilden, die durch mindestens drei gesättigte und/oder ungesättigte Zyklen oder durch mindestens drei Zyklen gebildet wird, von denen nur einer unter ihnen oder zwei unter ihnen aromatisch ist (sind) und untereinander ortho- oder ortho- und peri-kondensierte Systeme bilden.

3. Verfahren nach einem beliebigen der vorhergehenden Ansprüche, für die in der Formel (I):
• X', X", identisch oder unterschiedlich, ausgewählt sind aus dem Stickstoffatom, der C=O-Gruppe, der C=S-Gruppe und der C-OH-Gruppe;
• n 1 darstellt;
• m₄, m₇ und m₈ 1 darstellen; m₁, m₂, m₃, m₅ und m₆ 0 oder 1 darstellen;
• R₁, R₂, R₃, R₅ und R₆, identisch oder unterschiedlich, ausgewählt sind aus:
- einem Wasserstoffatom;
- einer verzweigten, linearen oder zyklischen (mono- oder polyzyklischen) Kohlenwasserstoffgruppe, die 1 bis 20 Kohlenstoffatome aufweist und eine oder mehrere Ungesättigtheiten in Form von Doppel- und/oder Dreifachbindung(en), vorzugsweise Methyl, Isobutyl, Phenyl, aufweisen kann;
- einem primären, sekundären oder tertiären Amin -NR'R", wobei R' und R", identisch oder unterschiedlich, eine C₁- bis C₁₀-Alkylgruppe, vorzugsweise eine C₁- bis C₆-Alkylgruppe, linear oder verzweigt, vorzugsweise Methyl, darstellen,
- einer Hydroxylgruppe;
• R₈ H darstellt und R₄, X" und R₇ eine heterozyklische Gruppe, vorzugsweise Pyrrolidin bilden; oder
• X', X", identisch oder unterschiedlich, ausgewählt sind aus dem Stickstoffatom, der C=O-Gruppe, der C=S-Gruppe und der C-OH-Gruppe;
• n 1 darstellt;
• m₂ bis m₆ 0 oder 1 darstellen; m₁, m₇ und m₈ 1 darstellen;
• R₁ bis R₆, identisch oder unterschiedlich, ausgewählt sind aus:
- einem Wasserstoffatom;
- einer verzweigten, linearen oder zyklischen (mono- oder polyzyklischen) Kohlenwasserstoffgruppe, die 1 bis 20 Kohlenstoffatome aufweist und eine oder mehrere Ungesättigtheiten in Form von Doppel- und/oder Dreifachbindung(en), vorzugsweise Methyl, Isobutyl, Phenyl, aufweisen kann;
- einem primären, sekundären oder tertiären Amin -NR'R", wobei R' und R", identisch oder unterschiedlich, eine C₁- bis C₁₀-Alkylgruppe, vorzugsweise eine C₁- bis C₆-Alkylgruppe, linear oder verzweigt, vorzugsweise Methyl, darstellen;
- einer Hydroxylgruppe;
• R₁, X' und R₇ eine Phenolgruppe bilden und R₈ H darstellt; oder
• X', X", identisch oder unterschiedlich, ausgewählt sind aus dem Stickstoffatom, der C=O-Gruppe, der C=S-Gruppe und der C-OH-Gruppe;
• n 2 darstellt;
• m₂, m₃, m₅, m₆ und m₈ 0 oder 1 darstellen; m₁, m₄ und m₇ 1 darstellen;
• R₂, R₃, R₅, R₆ und R₈, identisch oder unterschiedlich ausgewählt sind aus:
- einem Wasserstoffatom;
- einer verzweigten, linearen oder zyklischen (mono- oder polyzyklischen) Kohlenwasserstoffgruppe, die 1 bis 20 Kohlenstoffatome aufweist und einen oder mehrere Ungesättigtheiten in Form von Doppel- und/oder Dreifachbindung(en), vorzugsweise Methyl, Isobutyl, Phenyl, aufweisen kann;
- einem primären, sekundären oder tertiären Amin -NR'R", wobei R' und R", identisch oder unterschiedlich, eine C₁- bis C₁₀-vorzugsweise eine C₁- bis C₆-Alkylgruppe, linear oder verzweigt, vorzugsweise Methyl, darstellen;
- einer Hydroxylgruppe;
• R₁, X', X", R₇ und R₄ eine polyzyklische Gruppe bilden, die mindestens drei gesättigte und/oder ungesättigte Zyklen oder mindestens drei Zyklen aufweist, von denen einer oder zwei unter ihnen aromatisch sind und untereinander ortho- oder ortho- und peri-kondensierte Systeme, vorzugsweise die Phenanthrolingruppe, bilden.

4. Verfahren nach einem beliebigen der vorhergehenden Ansprüche, für das L ausgewählt ist aus

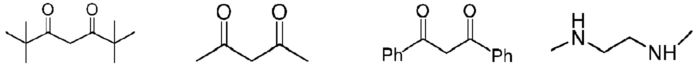

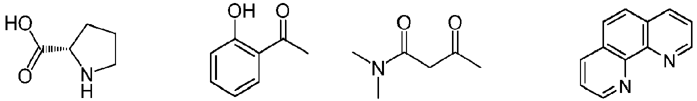

5. Verfahren nach einem beliebigen der Ansprüche 1 bis 4, für das X ausgewählt ist aus Brom oder Iod.

6. Verfahren nach einem beliebigen der Ansprüche 1 bis 5, für das R eine Gruppe mit induktiver Akzeptorwirkung ist, ausgewählt aus der NO₂-Gruppe, der CO₂Me-Gruppe, der CN-Gruppe, einem Halogenatom und der Methoxygruppe.

7. Verfahren nach einem beliebigen der Ansprüche 1 bis 5, für das X Brom ist und R eine Gruppe mit mesomerer Donatorwirkung ist, ausgewählt aus der Gruppe bestehend aus Phenyl, einer Hydroxygruppe, einer Methylgruppe, dem Fluoratom, dem Wasserstoffatom und einer Methoxygruppe, oder R mit dem Phenyl eine Naphthylgruppe bildet.

8. Verfahren nach einem beliebigen der vorhergehenden Ansprüche, für das das Kation M ausgewählt ist aus Kalium oder Cäsium.

9. Verfahren nach einem beliebigen der vorhergehenden Ansprüche, **dadurch gekennzeichnet, dass** die Hydroxilierungsreaktion in zwei Schritten gemäß dem folgenden Reaktionsschema durchgeführt wird: 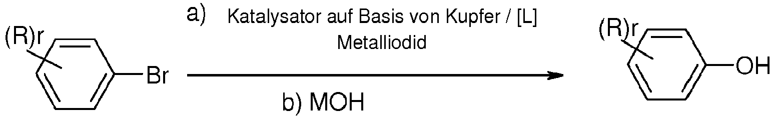 wobei
R in den Ansprüchen 1, 6 oder 7 definiert ist;
r im Anspruch 1 definiert ist;
L in den Ansprüchen 1 bis 4 definiert ist; und
M in den Ansprüchen 1 und 8 definiert ist;
der erste Schritt (a) einer nukleophilen Substitution des Brombenzens durch ein Metalliodid in Anwesenheit eines Katalysators auf Basis von Kupfer und eines Liganden entspricht,
der zweite Schritt der Hydroxylierungsreaktion in Anwesenheit eines Katalysators auf Basis von Kupfer und eines Liganden entspricht.

10. Verfahren nach Anspruch 9, für das das Metalliodid ausgewählt ist aus NaI, KI oder CsI.

11. Verfahren nach einem beliebigen der Ansprüche 9 oder 10, für das das Molverhältnis zwischen der Anzahl der Mole von Metalliodid und der Anzahl der Mole der Bromverbindung zwischen 0,1 und 4, vorzugsweise zwischen 1 und 3, variiert.

12. Verfahren nach einem beliebigen der vorhergehenden Ansprüche, für das die Katalysatoren auf Basis von Kupfer ausgewählt sind aus metallischem Kupfer, Kupfer(I)- oder Kupfer(II)-oxiden, Kupfer(I)- oder Kupfer(II)-hydroxiden, anorganischen oder organischen Salzen von Kupfer(I) oder Kupfer(II) und Komplexen von Kupfer(I) oder Kupfer(II) mit den üblichen Liganden.

13. Verfahren nach einem beliebigen der vorhergehenden Ansprüche, für das das Molverhältnis zwischen der Anzahl der Mole des Katalysators auf Kupferbasis und der Anzahl der Mole der halogenierten Arylverbindung zwischen 0,01 und 0,5 liegt.

14. Verfahren nach einem beliebigen der vorhergehenden Ansprüche, für das das Molverhältnis zwischen der Anzahl der Mole des Liganden L und der Anzahl der Mole der halogenierten Arylverbindung zwischen 0,001 und 0,9 variiert.

15. Verfahren nach einem beliebigen der vorhergehenden Ansprüche, für das das Molverhältnis zwischen der Anzahl der Mole von MOH und der Anzahl der Mole der bromierten Arylverbindung zwischen 0,1 und 5 variiert.

## Claims

1. Method for the hydroxylation of halogenated aryl compounds, comprising a hydroxylation reaction conducted at a temperature lower than 150°C, in the presence of a catalytic system consisting in a copper-based catalyst and a ligand L according to the following reaction scheme: 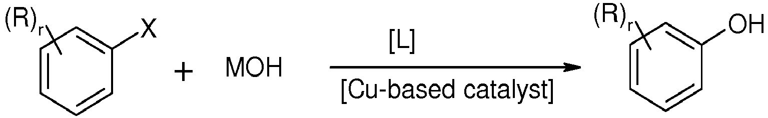
◆ R is selected in the group consisting of acceptor inductive effect groups chosen in the group consisting of NO₂ group, esters, CN group, halogen atom, alkoxy group, and donor mesomer effect groups chosen in the group consisting of phenyl group, hydroxyl group (OH), C1-C10, preferably C1-C6, linear or branched alkyl, halogen atom, hydrogen atom, alkoxy group or R for together with the phenyl a naphtyl group;
◆ r being between 0 and 5;
◆ X being a halogen atom;
◆ M being selected in the group consisting of alkaline or alkaline-earth cations;
◆ L represents a compound of formula (I) 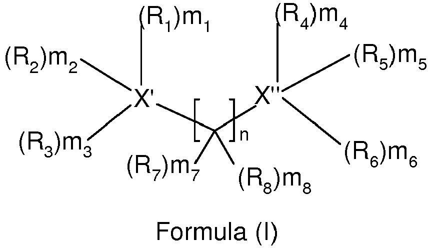 where
# X', X", the same or different, are selected in the group consisting of the nitrogen atom, the C=O group, the C=S goup and the C-OH group;
◆ m₁ to m₈, the same or different, represent 0 or 1;
◆ n represents 1 or 2;
◆ R₁, R₂, R₃, R₄, R₅, R₆, R₇ and R₈, the same or different, are selected in the group consisting of:
- a hydrogen atom;
- a branched, linear, monocyclic or polycyclic hydrocarbon group comprising 1 to 20 carbon atoms and possibly comprising one or unsaturations in the form of double and/or triple bond(s), preferably methyl, isobutyl, phenyl;
- a primary, secondary or tetriary -NR'R" amine, wherein R' and R", the same or different, represent a C₁ to C₁₀ alkyl group, preferably C₁ to C₆, linear or branched, preferably methyl;
- a hydroxyl group;
◆ or R₄, X" and R₇ form a heterocyclic group, preferably pyrrolidine;
◆ or R₁, X' and R₇ form a phenol group;
◆ or R₁, X', X", R₇ and R₄ form a polycyclic group formed of at least 3 saturated and/or unsaturated rings, or of at least 3 rings of which only one or two thereof is (are) aromatic and forming between them, ortho- or ortho- and peri-condensed systems, preferably the phenanthroline group.

2. The method according to claim 1, for which in formula (I):
◆ R₁, R₂, R₃, R₄, R₅, R₆, R₇ and R₈, the same or different, represent:
- a hydrogen atom;
- a C₁ to C₁₀ alkyl group;
- an aryl group; or
- a primary, secondary or tertiary -NR'R" amine wherein R' and R", the same or different, represent a hydrogen atom, a C₁ to C₁₀ alkyl group,
- a hydroxyl group;
◆ R₇ and R₈, the same or different, represent a hydrogen atom; or
◆ R₄, X" and R₇ form a heterocyclic group; or
◆ R₁, X' and R₇ form a phenol group; or
◆ R₁, X', X", R₇ and R₄ form a polycyclic group formed of a least 3 saturated and/or unsaturated rings, or of at least 3 rings of which only one or two thereof are aromatic and forming between them ortho- or ortho- and peri-condensed systems.
R₁, R₇ and X' form a phenol group;

3. The method according to any of the preceding claims for which in formula (I):
◆ X', X", the same or different are selected in the list consisting of the nitrogen atom, the C=O group, the C=S group and the C-OH group;
◆ n represents 1;
◆ m₄, m₇ and m₈ represent 1; m₁, m₂, m₃, m₅ and m₆ represent 0 or 1;
◆ R₁, R₂, R₃, R₅ and R₆ the same or different are selected in the list consisting of:
- a hydrogen atom;
- a branched, linear or cyclic (mono- or polycyclic) hydrocarbon group comprising 1 to 20 carbon atoms, and possibly comprising one or more unsaturations in the form of double and/or triple bond(s), preferably methyl, isobutyl, phenyl ;
- a primary, secondary or tertiary -NR'R" amine, wherein R' and R" the same or different represent a C₁ to C₁₀ alkyl group, preferably C₁ to C₆, linear or branched, preferably methyl;
- a hydroxyl group;
◆ R₈ represents H and R₄, X" and R₇ form a heterocyclic group, preferably pyrrolidine; or
◆ X', X", the same or different, are selected in the list consisting of the nitrogen atom, the C=O group, the C=S group and the C-OH group;
◆ n represents 1;
◆ m₂ to m₆ represent 0 or 1; m₁, m₇ and m₈ represent 1;
◆ R₁ to R₆, the same or different, are selected in the group consisting of:
- a hydrogen atom;
- a branched, linear or cyclic (mono- or polycyclic) hydrocarbon group comprising 1 to 20 carbon atoms and possibly comprising one or more unsaturations in the form of double and/or triple bond(s), preferably methyl, isobutyl, phenyl;
- a primary, secondary or tertiary -NR'R" amine, wherein R' and R" the same or different represent a C₁ to C₁₀ alkyl group, preferably C₁ to C₆, linear or branched, preferably methyl;
- a hydroxyl group;
◆ R₁, X' and R₇ form a phenol group and R₈ represents H; or
◆ X', X", the same or different, are selected in the list consisting of the nitrogen atom, the C=O group, the C=S group and the C-OH group;
◆ n represents 2;
◆ m₂, m₃ m₅, m₆ and m₈ represent 0 or 1; m₁, m₄ and m₇ represent 1;
◆ R₂, R₃, R₅, R₆ and R₈, the same or different, are selected in the list consisting of:
- a hydrogen atom;
- a branched, linear or cyclic (mono- or polycyclic) hydrocarbon group comprising 1 to 20 carbon atoms and possibly comprising one or more unsaturations in the form of double and/or triple bond(s), preferably methyl, isobutyl, phenyl;
- a primary, secondary or tertiary -NR'R" amine, wherein R' and R" the same or different represent a C₁ to C₁₀ alkyl group, preferably C₁ to C₆, linear or branched, preferably methyl;
- a hydroxyl group;
◆ R₁, X', X", R₇ and R₄ form a polycyclic group comprising at least 3 saturated and/or unsaturated rings, or at least 3 rings of which one thereof or two thereof are aromatic and form between them ortho- or ortho- and peri-condensed systems, preferably the phenanthroline group.

4. The method according to any of the preceding claims for which L is selected in the group consisting of:

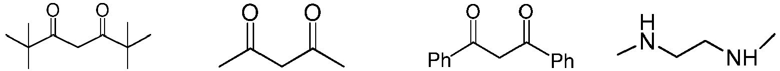

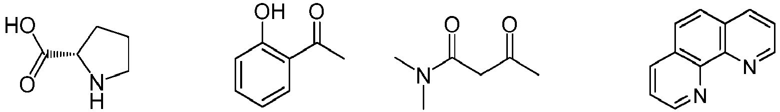

5. The method according to any of claims 1 to 4 for which X is selected from bromine or iodine.

6. The method according to any of claims 1 to 5 for which R is an acceptor inductive effect group selected in the list consisting of the NO₂ group, the CO₂Me group, the CN group, a halogen atom and the methoxy group.

7. The method according to any of claims 1 and 5 for which X is bromine and R is a donor mesomer effect group selected in the list consisting of phenyl, a hydroxyl group, a methyl group, the fluorine atom, the hydrogen atom and a methoxy group or R together with the phenyl forms a naphtyl group.

8. The method according to any of the preceding claims for which the cation M is selected in the list consisting of the salts of potassium or cesium.

9. The method according to any of the preceding claims, **characterized in that** the hydroxylation reaction is conducted in two steps according to the following scheme: 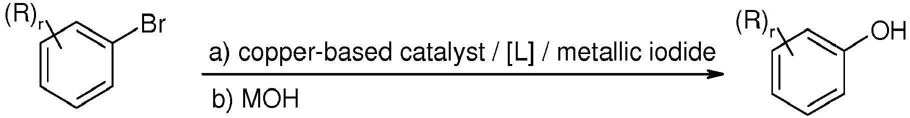 wherein:
R being defined under claims 1, 6 or 7;
r being defined under claim 1;
L being defined under claims 1 to 4; and
M being defined under claims 1 and 8;
the first step (a) corresponding to a nucleophilic substitution of bromobenzene by a metallic iodide in the presence of a copper-based catalyst and a ligand,
the second step corresponding to the hydroxylation reaction in the presence of a copper-based catalyst and a ligand.

10. The method according to claim 9 for which the metallic iodide is selected in the group consisting of Nal, Kl or Csl.

11. The method according to any of claims 9 or 10 for which the molar ratio between the number of moles of metallic iodine and the number of moles of brominated compound varies between 0.1 and 4, preferably between 1 and 3.

12. The method according to any of the preceding claims for which the copper-based catalysts are selected in the list consisting of metallic copper, copper (I) or copper (II) oxides, copper (I) or copper (II) hydroxides, the inorganic or organic salts of copper (I) or copper (II), and the complexes of copper (I) or copper (II) with usual ligands.

13. The method according to any of the preceding claims for which the molar ratio between the number of moles of copper-based catalyst and the number of moles of halogenated ayrl compound is comprised between 0.001 and 0.5.

14. The method according to any of the preceding claims for which the molar ratio between the number of moles of ligand L and the number of moles of halogenated aryl compound varies between 0.001 and 0.9.

15. The method according to any of the preceding claims for which the molar ratio between the number of moles of MOH and the number of moles of brominated aryl compound varies between 0.1 and 5.
